# EUROPEAN PATENT APPLICATION

(11) **EP 2 477 032 A1**
(43) Date of publication of application: **18.07.2012**
(21) Application number: 11250051.7
(22) Date of filing: 18.01.2011
(51) Int. Cl.: G01N 33/68

(54) **Measurement of anti-amyloid antibodies in human blood**

(71) Applicant: Baxter International Inc, Deerfield, IL 60015 (US); Baxter Healthcare SA, 8152 Glattpark (Opfikon) (CH); Cornell University, Ithaca, NY 14850 (US)
(72) Inventor: Relkin, Norman, Harrington Park, NJ 07646 (US); Szabo, Paul, Linden, NJ 07036 (US)
(74) Representative: HOFFMANN EITLE

(57) **Abstract**

The present invention provides improved immunoaffinity methods for detecting high avidity anti-amyloid antibodies present in a biological sample. In other aspects, the present invention provides methods for diagnosing diseases associated with amyloid proteins. Also provided by the present invention are methods for identifying candidates likely to respond to treatment comprising administration of an immunoglobulin preparation.

## Description

### Introduction

Misfolding and aggregation of the amyloid beta peptide (Aβ) is central to the pathogenesis of Alzheimer's disease (AD). The human immunoglobulin G (IgG) repertoire contains endogenous antibodies against the Aβ peptide that arise in the absence of vaccination or passive immunization. Endogenous anti-Aβ antibody activity has been detected in the blood of normal adults of various ages and patients with AD (Weksler et al., Exp Gerontol., 37:943-948 (2002); Hyman et al., Ann Neurol., 49:808-810.5 (2001); Mruthinti et al., Neurobiol Aging., 25:1023-1032 (2004); Nath et al., Neuromolecular Med., 3:29-39 (2003); Sohn et al., Frontiers in Bioscience., 14:3879-3883 (2009)). The human plasma-derived antibody preparation known as intravenous immunoglobulin (IVIG) has been reported to contain elevated levels of endogenous anti-amyloid antibodies and is under study as a potential treatment for AD (Dodel et al., J Neurol Neurosurg Psychiatry., 75:1472-1474 (2004); Hyman et al., Ann Neurol., 49:808-810.5 (2001); Mruthinti et al., Neurobiol Aging., 25:1023-1032 (2004)).

Published studies provide widely disparate estimates of the relative titers of anti-Aβ antibodies in AD patients versus aged normal controls. Initial studies of intact plasma specimens by standard ELISA methods ascribed lower titers of endogenous antibodies against Aβ monomers to AD patients than aged-matched non-demented controls (Weksler et al., Exp Gerontol., 37:943-948 (2002)). Subsequent studies reported equal or increased titers of circulating anti-Aβ monomers antibodies in AD patients (Mruthinti et al., Neurobiol Aging, 25:1023-1032 (2004)) based on ELISAs performed on plasma immunoglobulin eluted from Protein G columns at low pH (Akerström et al., J Biol Chem., 261:10240-10247 (1986)). The higher titers obtained by this method were hypothesized to reflect the presence of a pool of bound anti-amyloid antibodies that were undetected in assays of whole plasma and measurable when freed from antigen by acidification in the course of protein G purification. However in murine models, exposure of polyclonal IgG to low pH results in partial denaturation of antibodies and generates large artifactual increases in apparent anti-Aβ antibody titers (Li et al., BMC Neurosci., 8:22 (2007)). Assays that employ IgG isolated from human plasma by protein G chromatography and acid elution may therefore result in artifactually elevated measures of anti-amyloid activity.

The measurement of endogenous anti-Aβ antibody activity is further complicated by the existence of multiple classes of human antibodies that recognize linear as well as conformational neo-epitopes on aggregated forms of Aβ. Reports to date have identified endogenous human antibodies against Aβ fibrils (O'Nuallain et al., J Immunol., 176:7071-7078 (2006)), Aβ oligomers (Moir et al., J Biol Chem., 280:17458-17463 (2005); Relkin et al., Alzheimer's and Dementia, 3:S 196, X (2008); O'Nuallain et al., Biochemistry, 47:12254-12256 (2008)) and conformational epitopes on Aβ monomers (Baumketner A et al., Prot Sci., 15:420-428 (2006)). Other types of amyloid binding antibodies and even catalytic antibodies against Aβ have been reported (Taguchi H et al., J Biol Chem., 284:4714-4722 (2008)). Conventional ELISAs may under- or over-estimate total anti-amyloid activity if they fail to take into account the diversity of antibody types present as well as such phenomena as cross-reactivity and polyvalency.

Polyvalent antibodies to both foreign and self-antigens are part of the natural antibody repertoire of humans (Djoumerska et al., Scand J of Immunol., 61: 357-363 (2005)). Most polyvalent antibodies have germ line hypervariable regions, display lower affinity for their antigens compared to monovalent affinity-maturated antibodies and have more flexible antigen-binding sites. They are thought to serve as a defense mechanism against pathogens. An interesting feature of some polyvalent antibodies is that they can be generated from monovalent antibodies by mild disruptive treatments, *e.g.*, exposure to pH below about 4 or disruptive agents (Bouvet et al., J Autoimmun., 16:163-172 (2001); Dimitrov et al., Molec Immunol, 44:1854-1863 (2007)).

Methods to increase sensitivity and signal to noise of anti-Aβ antibody assays have been described, including a radio-immunoprecipitation assay (Brettschneider et al., Biol Psychiatry., 57:813-816 (2005)) and a novel peptide microarray method that was recently used to characterize endogenous human anti-Aβ antibodies in plasma as a function of age and AD (Britshgi et al., Proc. Natl. Acad. Sci. USA., 106:12145-12150 (2009)). Although the latter approach yielded improved signal to noise ratios compared to ELISA, its use of whole plasma may be problematic owing to the effects of other plasma proteins and macromolecules on anti-Aβ antibody measurements.

These and other factors that may confound measurements of endogenous anti-Aβ antibody levels have important implications for studies of treatment of AD by IVIG (intravenous immunoglobulin preparation). IVIG was first reported to contain anti-Aβ monomer antibodies (Dodel et al., J Neurol Neurosurg Psychiatry., 75:1472-1474 (2004)) at relatively low levels. The reported paucity of anti-Aβ antibodies brought into question the potential for IVIG to exert anti-amyloid effects in AD patients. Nevertheless, human clinical trials of IVIG in AD patients resulted in positive clinical outcomes as well as altered levels of Aβ in plasma and cerebrospinal fluid (Dodel et al., J Neurol Neurosurg Psychiatry., 75:1472-1474 (2004); Relkin et al., Neurobiol. of Aging, 30:1728-1736 (2009)). Although endogenous anti-amyloid antibody activity provided the original rationale for testing IVIG in AD, it is not known with certainty whether this is the major or exclusive mechanism of action of IVIG in this disease. The question of whether true endogenous anti-amyloid antibodies are present in human plasma at levels sufficient to exert biologically meaningful effects has yet to be answered.

As such, improved methods of assaying anti-amyloid activity in human plasma are needed to better understand the possible role of endogenous antibodies in the pathogenesis and treatment of AD and other amyloid-associated disorders. The present invention provides improved detection methods that address problems associated with the detection and quantitation of amyloid-specific antibodies, and consequently, with the detection of amyloid. The invention thus provides for more accurate diagnosis of diseases associated with amyloid proteins, patient selection for amyloid-related treatment, and other amyloid related applications.

### BRIEF SUMMARY OF THE INVENTION

The invention provides improved methods for preparing and assaying endogenous anti-amyloid antibodies in human plasma and intravenous immunoglobulin preparations. These novel methods show that human plasma contains polyvalent antibodies that bind to Aβ with lower avidity, as well as endogenous anti-amyloid antibodies with moderate to high avidity for Aβ and its aggregates.

In one aspect, the invention provides a method for detecting high avidity anti-amyloid antibodies present in a sample. In some embodiments, the method comprises the steps of (a) contacting the biological sample with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution comprising a chaotropic agent to dissociate at least one complex comprising an amyloid antigen and a low-affinity or non-specific antibody; and (c) detecting the presence or level of the remaining complexes.

In some embodiments, the biological sample is blood or a fraction thereof (*e.g.*, plasma or serum). In some embodiments, the biological sample is separated using thiophilic chromatography prior to step (a). In some embodiments, the biological sample is separated using thiophilic chromatography after step (b). In some embodiments, the thiophilic chromatography is used to remove non-immunoglobulin substances from the biological sample, to yield an enriched immunoglobulin preparation.

In a second aspect, the invention provides a method detecting a high avidity anti-amyloid antibody present in a biological sample, the method comprising the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; and (d) detecting the presence or level of the complex.

In some embodiments, the biological sample is blood or a fraction thereof (e.g., plasma or serum). In some embodiments, the complex formed between the amyloid antigen and the anti-amyloid antibody is washed with a solution containing a chaotropic salt prior to detecting the presence or level of the complex.

In a third aspect, the invention provides a method for identifying a subject who is a candidate for treatment of an amyloid-related disease or condition. In some embodiments, the method comprises the steps of (a) contacting a biological sample from the subject with a plurality of amyloid antigens under suitable conditions to form:
(i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific anti-amyloid antibody; (c) detecting the level of the remaining complexes; and (d) determining whether the subject would benefit from a treatment comprising administration of an immunoglobulin preparation by comparing the level of the anti-amyloid antibody to a control level. In one embodiment, the treatment comprises the administration of an immunoglobulin preparation.

In some embodiments of the aspects described herein, the control is from an individual or group of individuals that do not have an amyloid-related disease, such that an increased level of the remaining complex relative to control is indicative of a candidate for treatment. In some embodiments, the control is from an individual or group of individuals that do have an amyloid-related disease, such that a similar level of the remaining complex relative to control is indicative of a candidate for treatment. One of skill will understand how to select at least one appropriate control and interpret the results accordingly.

In a fourth aspect, the invention provides a method for identifying a subject who is a candidate for treatment of an amyloid-related disease or condition. In some embodiments, the method comprises the steps of (a) contacting the biological sample with a thiophilic resin to bind an anti-amyloid antibody present in the sample; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) determining whether the subject would benefit from a treatment comprising administration of an immunoglobulin preparation by comparing the level of the anti-amyloid antibody to a control level. In one embodiment, the treatment comprises the administration of an immunoglobulin preparation. As explained above, one of skill will understand how to select at least one appropriate control for diagnosing an amyloid-associated disorder and interpret the results accordingly.

In a fifth aspect, the invention provides a method for diagnosing a disease associated with an amyloid protein in a subject. In some embodiments, the method comprises the steps of (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) diagnosing the subject by comparing the level of the anti-amyloid antibody to a control level. In some embodiments, the invention provides methods for diagnosing Alzheimer's disease, or a likelihood of developing the disease, in a subject. As explained above, one of skill will understand how to select at least one appropriate control for diagnosing an amyloid-associated disorder and interpret the results accordingly.

In a sixth aspect, the invention provides a method for diagnosing a disease associated with an amyloid protein in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) diagnosing the subject by comparing the level of the anti-amyloid antibody to a control level. As explained above, one of skill will understand how to select at least one appropriate control for diagnosing an amyloid-associated disorder and interpret the results accordingly.

In a seventh aspect, the invention provides a method for providing a prognosis for the progression of a disease associated with an amyloid protein in a subject. In some embodiments, the method comprises the steps of (a) contacting a biological sample from the subject with a plurality of amyloid antigens under suitable conditions to form:
(i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) providing a prognosis for the progression of the disease by comparing the level of the anti-amyloid antibody to a control level. In some embodiments, the invention provides methods for providing a prognosis for the progression of Alzheimer's disease in a subject. As explained above, one of skill will understand how to select at least one appropriate control for providing a prognosis for an amyloid-associated disorder and interpret the results accordingly.

In an eighth aspect, the invention provides a method for providing a prognosis for the progression of a disease associated with an amyloid protein in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) providing a prognosis for the progression of the disease by comparing the level of the anti-amyloid antibody to a control level. In some embodiments, the invention provides methods for providing a prognosis for the progression of Alzheimer's disease in a subject. As explained above, one of skill will understand how to select at least one appropriate control for providing a prognosis for an amyloid-associated disorder and interpret the results accordingly.

In a ninth aspect, the invention provides a method for providing a prognosis for treatment of a disease associated with an amyloid protein, for example a known therapy or preventative strategy for AD. In some embodiments, the treatment can comprise administration of an immunoglobulin preparation such as IVIG in a subject. In some embodiments, the method comprises the steps of (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) providing a prognosis for treatment of the disease by comparing the level of the anti-amyloid antibody to a control level. In some embodiments, the invention provides methods for providing a prognosis for the treatment of Alzheimer's disease in a subject. As explained above, one of skill will understand how to select at least one appropriate control for diagnosing an amyloid-associated disorder and interpret the results accordingly.

In a tenth aspect, the invention provides a method for providing a prognosis for treatment of a disease associated with an amyloid protein, for example a known therapy or preventative strategy for AD. In some embodiments, the treatment can comprise administration of an immunoglobulin preparation such as IVIG in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) providing a prognosis for treatment of the disease by comparing the level of the anti-amyloid antibody to a control level. In some embodiments, the invention provides methods for providing a prognosis for the treatment of Alzheimer's disease in a subject. As explained above, one of skill will understand how to select at least one appropriate control for diagnosing an amyloid-associated disorder and interpret the results accordingly.

### BRIEF DESCRIPTION OF THE DRAWINGS

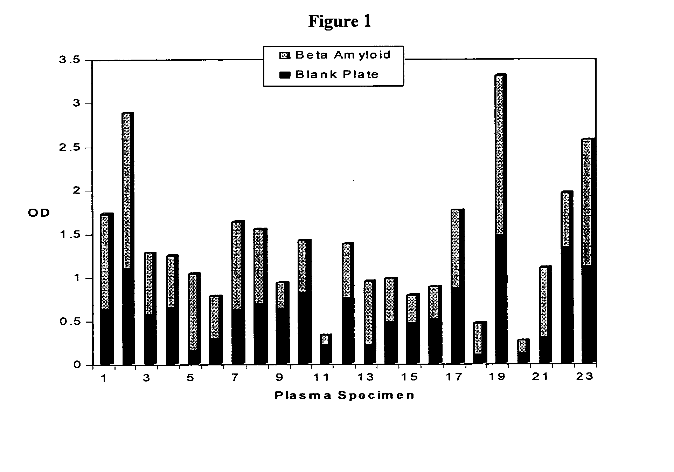

**Figure 1****.** IgG in human plasma binds significantly to empty ELISA wells as well as to Aβ. The relative binding to Aβ and blank plates is variable across individuals. Human plasma samples (n=23) were assayed by ELISA using plates with blank well and plates bearing Aβ monomer peptide. The resultant absorbance values (A₄₅₀ₙₘ) are plotted as histograms (blank wells, black; Aβ wells, grey) for each individual.

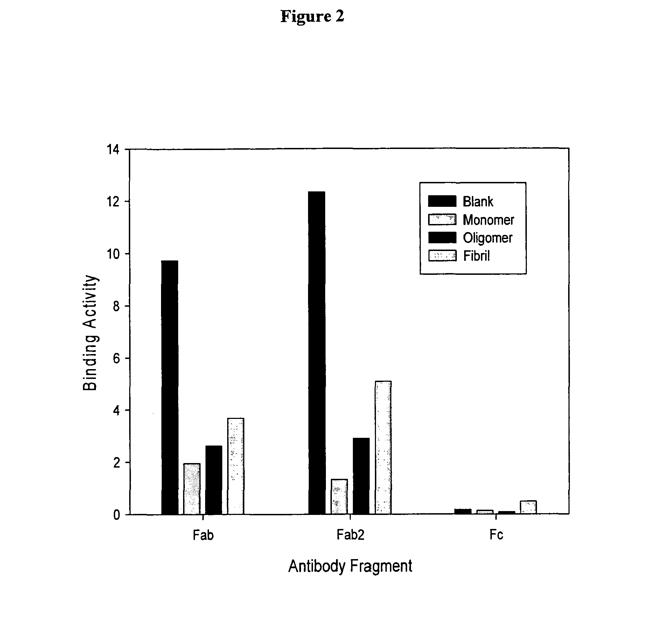

**Figure 2****.** Binding to empty wells occurs through the antigen binding region (F_{ab}) of the IgG molecule. Commercially available F_{ab}, F_{ab}2' and F_{c} fragments, isolated from pooled IgG of five individuals, were bound to blank and amyloid-bearing plates by standard ELISA methodology. Bound F_{ab} and F_{ab}2' fragments were detected using an anti-human kappa secondary antibody; F_{c} fragments using an anti-human IgG secondary antibody specific for the F_{c} portion of the molecule. Binding to blank plates is clearly mediated through the F_{ab} and F_{ab}2' regions of the IgG molecule rather than the F_{c} region.

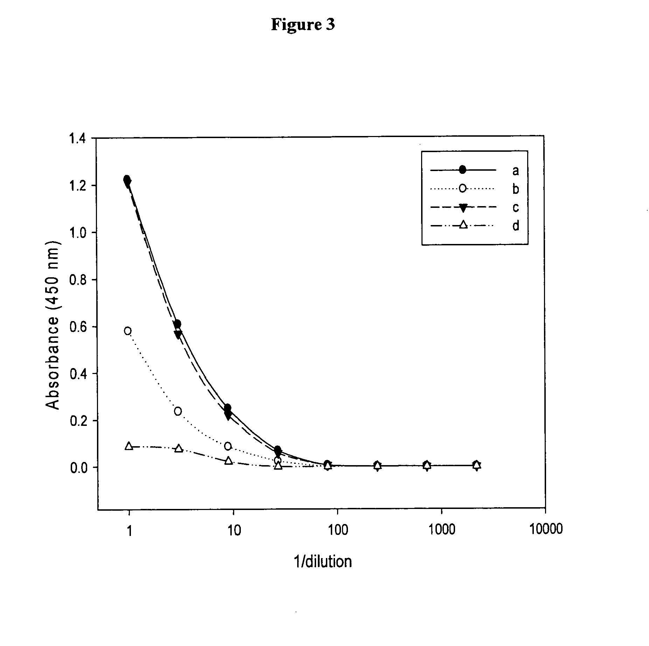

**Figure 3****.** Cross-reactivity of anti-Aβ antibodies in IVIG is exemplified by thyroglobulin's effects on the binding properties of anti-Aβ antibodies (a = Aβ plate, b = Aβ plate with thyroglobulin competition, c = thyroglobulin plate, d = thyroglobulin plate with thyroglobulin competition). ELISA measurements were made on using 3-fold serial dilutions of IVIG, starting at 1 mg/ml with and without the addition of 40 µg/ml thyroglobulin on plates bearing either thyroglobulin (triangles) or Aβ monomer peptide (circles). This level of thyroglobulin completely eliminates the binding of anti-thyroglobulin antibodies in IVIG to thyroglobulin plates and reduces the binding to Aβ plates by approximately 50%.

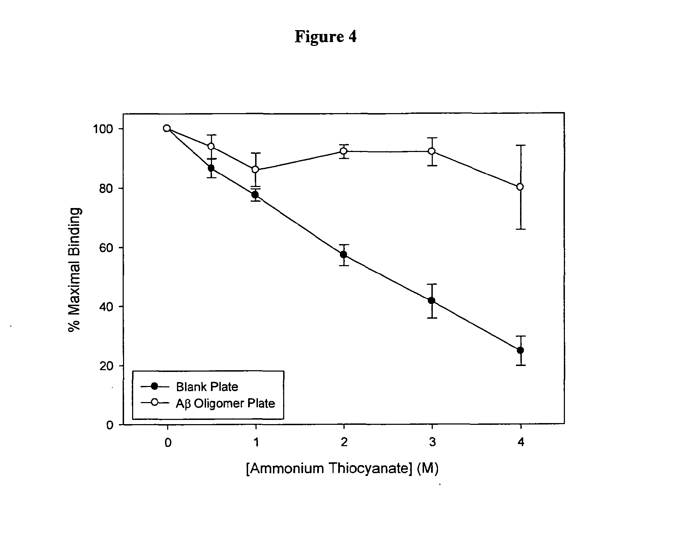

**Figure 4****.** The avidity of IVIG antibodies bound to ELISA plates bearing Aβ42 oligomers is greater than those bound to empty ELISA wells. To measure avidity, IVIG was bound to ELISA plates bearing Aβ42 oligomers or blank plates bearing no antigen. Quadruplicate wells were then incubated with increasing concentrations of the chaotropic salt ammonium thiocyanate, as indicated, and the amount of bound human antibody determined by standard ELISA methods. The results shown are the means and standard deviations of two independent determinations. Antibodies binding to Aβ oligomers have a greater avidity for their ligand than those that bind to empty wells.

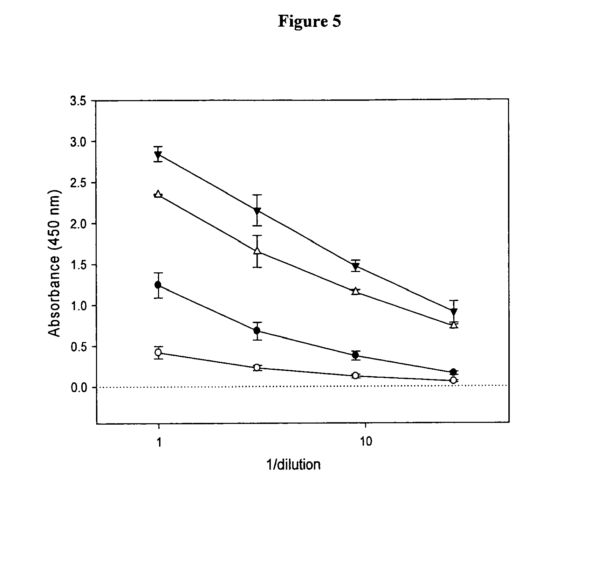

**Figure 5****.** 4M thiocyanate treatment improves the ratio of binding to wells containing Aβ42 oligomers vs binding to empty wells. Shown is a standard ELISA experiment in which the solid triangles indicate the binding to Aβ42 oligomer plates and the solid circles, the binding to blank plates. Open symbols indicate the same binding experiment followed by incubation with 4M thiocyanate. The ratio of Aβ42 oligomer to blank plate binding using purified human IgG (1 mg/ml) from control subjects is substantially increased from approximately 3, without 4M thiocyanate treatment, to between 7 and 10 following this treatment. Thus, treatment with the chaotropic agent permits the measurement of the higher avidity anti-Aβ oligomer antibodies in mixtures of purified human IgG, independent of low avidity antibodies that bind to empty ELISA wells.

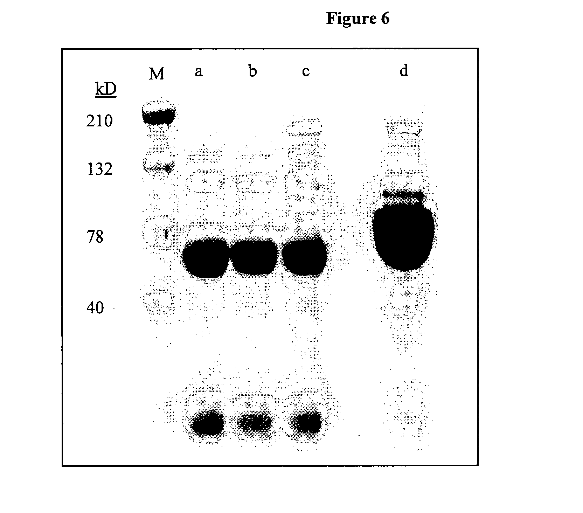

**Figure 6****.** Coomassie-stained 10% SDS:NuPAGE gel of protein samples reduced with β mercaptoethanol and loaded at equal mass shows IgG purified by chromatography on Thiophilic Adsorbant is nearly completely free of contaminating plasma proteins. The lanes are (a) IVIG, (b) IgG purified by chromatography on protein G, (c) IgG purified by chromatography on Thiophilic Adsorbant and (d) the original plasma sample. Also indicated are the molecular weight markers (M) and their sizes in kD.

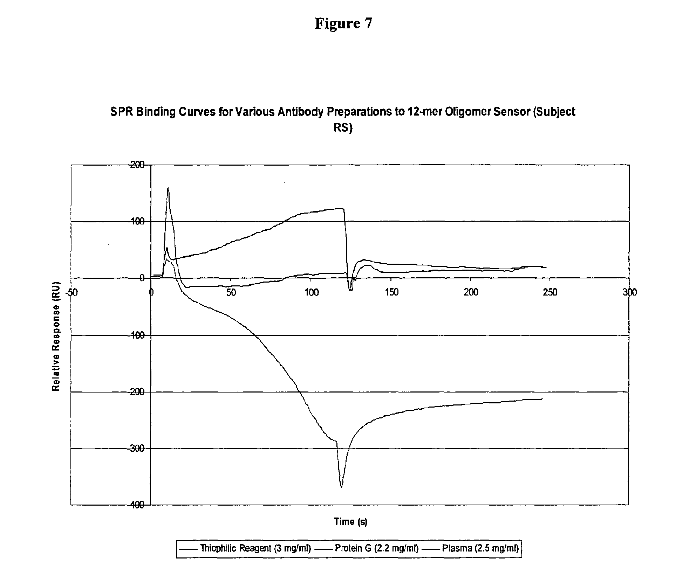

**Figure 7****.** Binding of whole plasma and antibodies isolated from plasma by Thiophilic or Protein G chromatography to an Aβ oligomer SPR sensor. SPR sensogram shows arbitrary response units (y-axis) as a function of time using an Aβ42 oligomer sensor. Sample injection was begun at 0 and ended at 125 seconds; SPR buffer flow was continued until 250 seconds before stripping and reconstitution of the sensor. The red line showing binding response of plasma is near zero as a result of interference from other plasma proteins and macromolecules. The bottom (pink) line shows the response to IgG isolated using protein G and acid elution. The result appears negative because of induced polyvalency and the resulting increased binding to the control sensor. The top (blue) line is the response of IgG prepared using Thiophilic Adsorbant. The initial 125 seconds reflect total anti-oligomer antibody binding activity (high and low affinity) whereas the portion of the sensogram after buffer wash (after 125 seconds) is principally attributable to antibodies with high affinity binding activity.

### DETAILED DESCRIPTION OF THE INVENTION

### Introduction

The diagnosis of Alzheimer's disease is difficult owing to the fact that the disease, especially in the early stages, presents with symptoms common to various other psychological conditions. Definitive diagnosis may be achieved by the visualization of Aβ tangles and plaques formed in the brain. Likewise, other amyloid-related diseases and conditions are commonly diagnosed with the help of tissue biopsies, on which amyloid deposits can be stained using the Congo red procedure (Linke, R.P. (2006) "Protein misfolding, aggregation and conformational diseases" (V.N. Uversky and A.L. Fink, eds.), Protein Reviews, Volume 4, (M.Z. Atassi, ed.); Chapter 11.1, pp. 239-276; Springer). However, due to the risks and complications involved with performing biopsies of organs such as the brain and heart, this is not a practical solution for the diagnosis of various amyloid-related diseases, including, Alzheimer's disease, Creutzfeldt-Jakob disease, and cardiac amyloidosis.

Advantageously, the present invention provides non-invasive methods for the diagnosis, or for aiding in the diagnosis, of amyloid-related diseases, through the specific detection and/or quantification of amyloid proteins in blood and plasma samples. In certain embodiments, the methods provided herein are useful for aiding in the diagnosis of amyloid-related disorders, wherein the method further comprises using a second diagnostic method. For example, the methods provided herein may be used in combination with other diagnostic tools to provide a diagnosis or prognosis for an amyloid protein related disorder. In one embodiment, the methods provided herein can be used in combination with tests and evaluations used to diagnose Alzheimer's disease, which include without limitation, cognitive tests, psychosocial histories, mental state examinations, neuropsychological testing, familial history, CT scans, electroencephalography (EEG), Positron Emission Tomography (PET) scans, Single Photon Emission Computed Tomography (SPECT) Scans, Magnetic Resonance Spectroscopy Imaging (MRSI), tests that rule out other disorders (e.g., physical exam, chest X-ray, magnetic resonance imaging, electrocardiogram (ECG)), and the like. These methods overcome significant hurdles associated with the detection of these proteins, such as the non-specific binding of low avidity and/or polyvalent antibodies to amyloid proteins.

Similarly, the present invention also provides methods for aiding in treatment decisions. The non-invasive methods for detecting high avidity anti-amyloid antibodies provided herein allow for the assignment and/or administration of a therapy for an amyloid-related disorder. For example, the present invention provides methods for identifying a patient likely to benefit from a treatment for an amyloid-related disorder, diagnosing an amyloid-related disorder, providing a prognosis for an amyloid-related disorder. Accordingly, in certain embodiments, the methods further comprise a step of assigning and/or administrating a treatment for an amyloid-related disorder (e.g., IVIG administration or other known therapy) to the subject.

The human immunoglobulin G (IgG) repertoire contains endogenous antibodies against beta amyloid (Aβ) that arise in the absence of immunization. These antibodies can be used for diagnosis and treatment of amyloid-related diseases, such as Alzheimer's disease (AD). The present invention addresses problems that complicate the measurement of these antibodies in plasma and pool immunoglobulin preparations such as IVIG (immunoglobulin preparation from pooled plasma). These problems include relatively high background binding to empty ELISA wells, assay interference attributable to other plasma proteins, and the confounding effects of polyvalency. Addressing the confounding issues that call antibody specificity into question also improves the reliability of amyloid detection using amyloid-specific antibodies.

The present invention provides, among other aspects, methods that improve the measurement of anti-Aβ antibodies in plasma. One factor that can confound accurate of anti-Aβ antibodies measurements is the presence of polyvalent IgG in human plasma that binds to blank ELISA plates (as well as unfilled areas on Aβ plates). Blank plate binding is variable across individuals and not effectively eliminated by standard ELISA blocking agents or background subtraction.

Quantification of anti-Aβ antibodies in human plasma or serum has been carried out in most instances on ELISA plates bearing either monomeric or aggregated (oligomeric or fibrillar) Aβ peptide. Plasma samples from normal human donors assayed by this method bind significantly to blank ELISA wells (wells not bearing any added antigen). Extensive, non-specific blank plate binding occurs despite the use of traditional blocking agents such as skim milk, fetal calf serum, albumin and commercial blocking preparations (Klaver et al., J. Neurosci. Meth. 187, 263-269).

The presence of antibodies that have the capacity to bind to blank ELISA wells complicates measurement of anti-Aβ oligomer antibodies in individuals since the observed absorbance values obtained for empty wells can be comparable or even greater than those obtained for amyloid-bearing plates (Figure 1).

It is demonstrated herein that blank plate binding can be significantly reduced by exposure of the antigen-antibody complex to a chaotropic salt solution of suitable molarity to dislodge weakly bound polyvalent antibodies without significantly reducing the binding of specific anti-Aβ antibodies.

Another potential source of inaccuracy in anti-Aβ antibody measurements is binding interference by other proteins and macromolecules present in whole plasma. Isolation of IgG from plasma can eliminate this interference but exposure to low pH during isolation by Protein G chromatography generates polyvalency that artifactually inflates measured levels of anti-Aβ antibodies.

Using the improved methods provided herein, surface plasmon resonance (SPR) measurements confirm that human blood (and IVIG) contains polyvalent antibodies with relatively low avidity for Aβ, as well as antibodies with moderate to high avidity for conformational epitopes on Aβ monomers and aggregates (*e.g.*, oligomers, filaments).

It is demonstrated herein that more accurate binding measurements can be obtained by avoiding exposure of IgG to denaturing conditions during isolation. Assays of IgG subjected to thiophilic chromatography yield titers of anti-amyloid antibodies that are essentially unchanged from the starting material, indicating that no artifactual polyvalency has been created. In contrast, IgG subjected to acid-elution from a Protein G column artifactually gains as much as an order of magnitude or more of anti-amyloid activity. IgG produced by passage over Thiophilic absorbant columns can include antibodies bound to antigen. Thus, the measured titer is that of free antibody. Thiophilic chromatography removes a majority of the plasma proteins, some of which interfere with measurements of anti-amyloid antibodies in whole plasma. Measurements such as the microarray method using whole plasma may be confounded the presence of other proteins that can vary across individuals of different ages and disease states (Britshgi M et al., Proc. Natl. Acad. Sci. USA., 106:12145-12150 (2009)).

As explained above, the complexities involved in measuring human anti-amyloid antibodies have created uncertainty as to whether endogenous amyloid-specific IgG actually exists or if such antibodies represent naturally-occurring or artifactually-induced polyvalent antibodies. The results presented herein show that endogenous human anti-amyloid antibodies can indeed be isolated from IVIG by affinity chromatography. These antibodies show specificity for either linear or conformational epitopes of beta amyloid with binding constants in the hundreds of namomolar range. This is within an order of magnitude of the Kd measured for murine monoclonal anti-amyloid antibodies using the same SPR sensors, and represents considerably higher binding avidity than would be expected were this activity to have arisen from a non-specific, polyvalent antibody-antigen interaction.

Moreover, anti-fibril antibodies isolated by sequential passage over beta amyloid and IAPP fibril affinity columns show less cross-reactivity with monomeric and oligomeric amyloid antigens, as well as blank plates. These results suggest that conformational neo-epitopes that differ across stages of amyloid assembly are recognized by the human IgG repertoire.

It is shown herein that high avidity endogenous anti-amyloid antibodies represent less than 0.1 % of the total mass of plasma-derived human IgG. The greater amyloid binding capacity of IVIG relative to individual plasma samples could reflect the clonal diversity of IVIG inherent in its derivation from thousands of plasma donors. Another source of increased anti-Aβ activity could be less specific binding that arises as a consequence of the chemical and physical processes involved in IVIG production.

Naturally-occurring auto-antibodies can regulate the uptake of self antigens and promote specific CD4+ T cell responses (Nielsen, Eur J Immunol., 31:2660-2668 (2001)). Polyvalent, anti-Aβ auto-antibodies and/or those generated by exposure to mildly denaturing conditions may have effects on the levels of Aβ peptide in circulation or the central nervous system. Thus, endogenous anti-Aβ antibodies may be responsible for the therapeutic benefits of IVIG in AD patients.

Advantageously, the methods provided herein allow for the specific detection and quantitation of high avidity anti-amyloid antibodies. Specifically, the methods provided herein allow for the detection and quantification of a heterogenous population of antibodies that bind an amyloid protein of interest with high affinity, *e.g.*, with a Kd in the range of a monoclonal antibody that was specifically raised against the amyloid protein of interest.

### Definitions

As used herein, a "chaotropic agent" refers to a chemical that destabilizes the three-dimensional structure of proteins. In certain embodiments, a chaotropic agent can refer to an ionic chaotrope (*e.g.*, a chaotropic ion or chaotropic salt) or alternatively to a non-ionic chaotrope. Non-limiting examples of chaotropic salts include: guanidinium salts, *e.g.*, guanidinium chloride, guanidinium nitrate, guanidinium thiocyanate; thiocyanate salts, *e.g.*, ammonium thiocyanate, potassium thiocyanate, sodium thiocyanate, lithium thiocyanate, calcium thiocyanate, guanidinium thiocyanate; perchlorate salts, *e.g.*, ammonium perchlorate, sodium perchlorate, lithium perchlorate, magnesium perchlorate, calcium perchlorate; iodide salts, *e.g.*, ammonium iodide, potassium iodide, sodium iodide, lithium iodide, magnesium iodide, calcium iodide; chlorate salts, *e.g.*, sodium chlorate, lithium chlorate, magnesium chlorate, calcium chlorate; and the like. Non-ionic chaotropes include, without limitation, urea and thiourea.

"Thiophilic," in reference to chromatography and similar affinity-based methods, refers to affinity selection based on a sulfur-containing ligand for a protein, in particular, for immunoglobulins, in high concentrations of certain salts. The protein can be eluted by removal of the salts. Thiophilic affinity chromatography (TAC) has a broad specificity for immunoglobulins of different classes and species, and offers advantages over Protein A or G purification because of the relatively mild conditions, reduced cost of materials, and broad range of specificity. Exemplary salts for use with TAC include ammonium, sodium, and potassium sulfate, though others are commonly used. TAC reagents and gels are commercially available, *e.g.*, from Millipore®. For a review, see Matejtschuk (2004) Methods in Mol. Biol. 244:195-204.

The terms "antibody," "immunoglobulin," and like terms refer to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively.

An exemplary immunoglobulin (antibody) structural unit is composed of two pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively. The C terminal ends of each heavy chain are disulfide bonded together, and form the constant region of the antibody.

The term "specifically bind" refers to a molecule (*e.g.*, targeting agent, antibody) that binds to a target (antigen, epitope, etc.) with at least 2-fold greater affinity than non-target compounds, *e.g.*, at least 4-fold, 5-fold, 6-fold, 7-fold, 8-fold, 9-fold, 10-fold, 20-fold, 25-fold, 50-fold, or 100-fold greater affinity.

The terms "antigen," "analyte," "antibody target," etc. are used interchangeably herein. In some cases, the site of interaction of an antibody with its target is defined, *e.g.*, the targeted site, binding interaction site, or "epitope." In some cases, the epitope is a three-dimensional moiety. Thus, for example, where the target is a protein, the epitope can be comprised of consecutive amino acids, or amino acids from different parts of the protein that are brought into proximity by protein folding (*e.g.*, a discontinuous epitope). The same is true for other types of target molecules that form three-dimensional structures.

Thus, the term "amyloid antigen" can refer to an amyloid monomer, oligomer, or fibril, or can instead refer to an amyloid fragment or single epitope. One of skill will appreciate that a given amyloid protein or aggregate will typically comprise a plurality of amyloid antigens or epitopes.

Antibody affinity is the strength of the reaction between a single antigenic determinant and a single combining site on the antibody. It is the sum of the attractive and repulsive forces operating between the antigenic determinant and the combining site of the antibody. Affinity is typically expressed in terms of a dissociation constant (K_{D}, Kd, etc.). The higher the affinity of an antibody for an antigen, the lower the Kd.

As used herein, the term "low affinity" and "non-specific" are relative terms. The cutoffs between high, medium, low, and no affinity can be set by the user depending on the desired stringency, and the range of affinity measurements obtained in a particular individual. Typically, a high affinity antibody will have a Kd less than about 10 nM. In certain embodiments, a high affinity antibody will have a Kd less than about 10 nM or less than about 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 50 pM, 25 pM, 10 pM, 5 pM, 1 pM, or lower. Typically, a low affinity antibody will have a Kd greater than about 100 nM. In certain embodiments, a low affinity antibody will have a Kd greater than about 100 nM or greater than about 200 nM, 300 nM, 400 nM, 500 nM, 600 nM, 700 nM, 800 nM, 900 nM, 1 µM, 2 µM, 3 µM, 4 µM, 5 µM, 6 µM, 7 µM, 8 µM, 9 µM, 10 µM, or greater.

Avidity is a measure of the overall strength of binding of an antigen with many antigenic determinants and multivalent antibodies. Avidity is influenced by both the valence of the antibody and the valence of the antigen. Avidity, however, is more than the sum of the individual affinities. That is, affinity refers to the strength of binding between a single antigenic determinant and an individual antibody combining site whereas avidity refers to the overall strength of binding between multivalent antigens and antibodies. In embodiments of the methods provided herein, an anti-amyloid antibody being detected has high avidity for the target amyloid protein. In certain embodiments, an anti-amyloid antibody being detected has both high avidity and high affinity for a target amyloid epitope. In other embodiments, an anti-amyloid antibody being detected has high avidity for a target amyloid protein yet low affinity for an individual amyloid epitope.

As used herein, an "amyloid protein" refers to a polypeptide capable of polymerizing to form a cross-beta structure *in vivo* or *in vitro.* These proteins are abnormally deposited in organs and/or tissues of individuals suffering from conditions characterized by amyloidosis (*e.g.*, Alzheimer's disease, Parkinson's disease, Creutzfeldt-Jakob disease, *etc.*). Accordingly, an anti-amyloid antibody is an antibody that specifically recognizes an amyloid protein. Anti-amyloid antibodies specifically recognize one or more oligomeric state of a particular amyloid protein, for example, an monomeric, dimeric, or oligomeric amyloid protein. Non-limiting examples of amyloid proteins include Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), and Immunoglobulin light chain (AL). Any of these or other amyloid proteins can be detected by using the methods provided herein.

As used herein, a "biological sample" refers to a cell or population of cells or a quantity of tissue or fluid from a subject. Most often, the sample has been removed from an animal, *e.g.*, a human. "Biological sample" includes blood and blood fractions (*e.g.*, plasma, serum, etc.), other biological fluids (*e.g.*, urine, saliva, lymph fluids, Cerebrospinal fluid (CSF), *etc*.), sections of tissues such as biopsy and autopsy samples, frozen sections taken for histologic purposes, lymph tissue, cultured cells, and the like.

As used herein, the term "about" denotes an approximate range of plus or minus 10% from a specified value. For instance, the language "about 20%" encompasses a range of 18-22%. As used herein, about also includes the exact amount. Hence "about 20%" means "about 20%" and also "20%." As used herein, "about" refers to a range of at or about the specified value.

The terms "dose" and "dosage" are used interchangeably herein. A dose refers to the amount of active ingredient given to an individual at each administration. The dose will vary depending on a number of factors, including frequency of administration; size and tolerance of the individual; severity of the condition; risk of side effects; and the route of administration. One of skill in the art will recognize that the dose can be modified depending on the above factors or based on therapeutic progress. The term "dosage form" refers to the particular format of the pharmaceutical, and depends on the route of administration. For example, a dosage form can be in a liquid, e.g., a saline solution for injection.

A "therapeutically effective" amount or dose or "sufficient/effective" amount or dose, is a dose that produces effects for which it is administered. The exact dose will depend on the purpose of the treatment, and will be ascertainable by one skilled in the art using known techniques (*see*, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

As used herein, the term "prevent" refers to a decreased likelihood or reduced frequency of cognitive symptoms or amyloid plaques in a patient. The prevention may be complete or partial, such that fewer symptoms are observed in a patient than would have occurred without the present invention.

The terms "therapy," "treatment," and "amelioration" refer to any reduction in the severity of symptoms or amount of amyloid aggregation, or improvement in cognitive function. As used herein, the terms "treat" and "prevent" are not intended to be absolute terms. Treatment can refer to any delay in onset, amelioration of symptoms, improvement in patient survival, increase in survival time or rate, etc. The effect of treatment can be compared to an individual or pool of individuals not receiving the treatment.

A "control" sample or value refers to a sample that serves as a reference, usually a known reference, for comparison to a test sample. For example, a test sample can be taken from a patient of unknown amyloid status and compared to control samples from a patient diagnosed with amyloidosis (*e.g.*, Alzheimer's disease, Parkinson's disease, Creutzfeldt-Jakob disease, *etc*.) and/or an individual not suffering from amyloidosis or known to have low anti-amyloid antibody titre. A control can also represent an average value gathered from a population of similar individuals, e.g., Alzheimer's disease patients, Parkinson's disease patients, Creutzfeldt-Jakob disease patients, *etc.* with a similar medical background, or of the same age, weight, *etc.* A control value can also be obtained from the same individual, e.g., from an earlier-obtained sample, prior to symptoms, or before or at different therapeutic time points. In some cases, controls can include comparisons within an individual or between individuals, *e.g.*, comparison of anti-amyloid antibody titres with the titre of one or more known antibodies.

One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

### Alzheimer's Disease and Other Amyloid-Related Disorders

Amyloid proteins and abnormal protein aggregates play a role in a number of different diseases and conditions. The invention provides methods for detecting specific high-avidity auto-antibodies against various forms of amyloid proteins or certain protein aggregates which can be useful for diagnosis and determining a course of therapy.

Amyloid related disorders include not only Alzheimer's Disease (AD), but also Parkinson's Disease, Familial Amyloid Polyneuropathy (FAP), various forms of amyloidosis (*e.g.*, senile systemic, Finnish type, Icelandic type, leptomeningeal, familial visceral, cutaneous, dialysis-related, etc.), and aoric medial amyloid. Additional disorders related to abnormal protein aggregation include Type II diabetes mellitus, transmissible spongiform encephalopathy, Creutzfeldt-Jakob disease, Huntington's Disease, medullary thyroid carcinoma, atherosclerosis, rheumatoid arthritis, prolactinomas, Frontotemporal Dementias, Down's syndrome. Lewy body disease, and similar diseases

Accordingly, the invention can be used to detect and/or isolate auto-antibodies against specific forms of amyloid and amyloid aggregates found in amyloid-related disorders. In addition, the methods disclosed herein can be applied to detection of antibodies against abnormal protein aggregates formed from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), proteins having polyQ repeats, Tau protein (Tau), and other amyloid proteins.

Detection of specific auto-antibodies, *e.g.*, for Aβ fibrils in the case of AD and Parkinson's, can be used in diagnosis or risk factor assessment. The diagnostic methods of the invention can be applied to individuals considered at risk for developing an amyloid-related disorder, *e.g.*, based on age, family history, cognitive symptoms, etc., as can be determined by one of skill in medicine.

Risk factors and symptoms of amyloid-related disorders will be best determined by a skilled medical practitioner. Risk of developing these disorders increases with age, and correlate with family history. In many cases, an absolutely definitive diagnosis is considered unfeasible. Observable symptoms of AD include disruptive memory loss, differences in ability to solve problems, confusion as to time or place, trouble completing routine tasks, social withdrawal, and mood changes. These correlate with physical manifestations of the disease such as increased amyloid plaque formation and overall decrease in brain volume.

### Detection and Isolation of Anti-Amyloid Immunoglobulins

Low levels of auto-antibodies can be found in the blood of individuals that have not been inoculated with a given antigen. For example, plasma preparations pooled from a number of individuals include a small fraction of IgG antibodies that bind to amyloid proteins such as Aβ. As explained herein, inaccurate methods determining antibody specificity and harsh methods of antibody isolation have caused uncertainty as to whether the amyloid-specific antibodies actually exist, or instead represent artifacts or non-specific interactions.

The present invention provides methods of detecting and/or isolating antibodies that are specific for various forms of amyloid, including monomeric, oligomeric, and fibrillar amyloid proteins (*i.e.*, Aβ). For example, the small population of Aβ-specific IgGs can be isolated from a biological sample by removing low-affinity and non-specific antibodies using a mild chaotropic wash. In some embodiments, the methods include first separating IgG from other proteins in the sample using thiophilic affinity-based methods, and removing low-affinity and non-specific antibodies using a mild chaotropic wash. These steps can be performed in any order. Typically, the biological sample is blood plasma or serum.

One of skill in the art will appreciate that the chaotropic agent should be present in an amount sufficient to disrupt the weak binding interactions of low affinity or non-specific antibodies without significantly disrupting the binding specificity of high-affinity antibodies. For example, while urea is a chaotropic agent, a high concentration of urea can generate polyvalency. This is not fatal to the detection methods of the invention, as any amyloid-specific affinity generated by the chaotropic agent will typically be low.

The antibodies can be detected using standard methods, *e.g.*, using a detectably labeled antibody binding agent such as an anti-human secondary antibody, antigen (*e.g.*, Aβ), Protein A, Protein G, etc. Such methods include ELISAs, Western blots, immunofluorescence, and other protein detection methods known in the art (*see*, *e.g.*, U.S. Patents 4,366,241; 4,376,110; 4,517,288; and 4,837,168). For a review of the general immunoassays, see also METHODS IN CELL BIOLOGY, VOL. 37, Asai, ed. Academic Press, Inc. New York (1993); BASIC AND CLINICAL IMMUNOLOGY 7TH EDITION, Stites & Terr, eds. (1991). Immunological binding assays (or immunoassays) typically utilize a ligand (*e.g.*, amyloid, Aβ, or a particular form of Aβ) to specifically bind to and often immobilize an antibody.

Immunoassays also often utilize a labeling agent to specifically bind to and label the binding complex formed by the ligand and the antibody. The labeling agent can itself be one of the moieties comprising the antibody/analyte complex, *e.g.*, detectably labeled analyte. Alternatively, the labeling agent may be a third moiety, such as another antibody, that specifically binds to the antibody/analyte complex.

A competitive assay can be used, wherein the labeling agent is a second anti-amyloid antibody bearing a label. The two antibodies then compete for binding to the immobilized amyloid antigen. Alternatively, in a non-competitive format, the anti-amyloid antibody lacks a label, but a second antibody specific to antibodies of the species from which the anti-amyloid antibody is derived, e.g., human, and which binds the anti-amyloid antibody, is labeled.

Other proteins capable of specifically binding immunoglobulin constant regions, such as Protein A or Protein G can also be used as the label agent (*see*, generally Kronval, et al., J. Immunol. 111:1401-1406 (1973); and Akerstrom, et al., J. Immunol. 135:2589-2542 (1985)).

Throughout the assays, incubation and/or washing steps may be required after each combination of reagents. Incubation steps can vary from about 5 seconds to several hours, *e.g.*, from about 5 minutes to about 24 hours. However, the incubation time will depend upon the assay format, antibody, volume of solution, concentrations, and the like. Usually, the assays will be carried out at ambient temperature, although they can be conducted over a range of temperatures, such as 10°C to 40°C.

If desired, the user can set a cutoff range for high, medium, and low affinity/avidity, and detect antibodies that fall within these ranges. Affinity assays can be used to determine the dissociation constant (Kd) of the antibody. The phrase "dissociation constant" refers to the affinity of an antibody for an antigen. Specificity of binding between an antibody and an antigen exists if the dissociation constant (K_{D} = 1/K, where K is the affinity constant) of the antibody is < 1µM, with increasing specificity and affinity antibodies often having a K_{D} < 100 nM or < 10 nM. K_{D} = [Ab-Ag]/[Ab][Ag] where [Ab] is the concentration at equilibrium of the antibody, [Ag] is the concentration at equilibrium of the antigen and [Ab-Ag] is the concentration at equilibrium of the antibody-antigen compiex. Typically, the binding interactions between antigen and antibody include reversible non-covalent associations such as electrostatic attraction, Van der Waals forces and hydrogen bonds. This method of defining binding specificity applies to single heavy and/or light chains, CDRs, fusion proteins or fragments of heavy and/or light chains.

The resulting population of Aβ-specific antibodies is heterogenous, but binds to Aβ with high affinity, *e.g.*, with a Kd in the range of a monoclonal antibody that was specifically raised against Aβ.

### Therapies for Amyloid Related Disorders and Alzheimer's Disease

Treatments for amyloid-related diseases and conditions, such as AD, are typically focused on cognitive and mood symptoms of the disease. Early treatment and prevention regimes include physical and social activity, memory games, and puzzle and problem solving. Pharmaceutical therapies for symptomatic individuals include cholinesterase inhibitors (to address reduced acetylcholine), partial glutamate antagonists (*e.g.*, Memantine), and psychiatric drugs (*e.g.*, antipsychotics, sleep aides, anxiolytics, and beta-blockers). Cholinesterase inhibitors include Aricep®t (donepezil hydrochloride), Exelon® (rivastigmine), Razadyne® (galantamine), and Cognex® (tacrine).

It has also been observed that pooled immunoglobulin preparations can be effective for improving AD symptoms. Such preparations, called IVIG (intravenous immunoglobulin), are prepared as described, e.g., in USSN 12/789,345 (filed May 27, 2010) and USSN 12/789,365 (filed May 27, 2010). Methods of treating AD, Parkinson's, and other protein aggregation disorders using IVIG are disclosed in US Pub. Nos. 2009/0148463 and 2009/0221017.

Briefly, IVIG is formed by pooling intravenous blood from more than one individual, separating the plasma fraction, and enriching for IgG immunoglobulin using a combination of chromatography, ultrafiltration, and diafiltration. IVIG is typically administered by intravenous infusion.

### Methods for the Detection of Anti-Amyloid Antibodies

In one aspect, the present invention provides improved methods for detecting anti-amyloid antibodies in a biological sample. For example, methods are provided herein for the measurement of high avidity anti-amyloid antibodies present in the blood of a mammal. In one embodiment, the present invention provides methods for the accurate quantification of antibodies against aggregated or non-aggregated amyloid proteins present in biological fluids. These antibodies are emerging as significant tools for prediction, diagnosis and treatment of human amyloidosis diseases including Alzheimer's disease (AD) and other neurodegenerative disorders. Antibodies may also develop in other mammalian species particularly those used as models of these diseases. As such, the present invention improves the quantification and characterization of antibodies against aggregated amyloid proteins as well as unaggregated amyloid monomers when the level of monomers is low in the fluid being studied.

Thus, the present invention provides methods of detecting anti-amyloid antibodies, *e.g.*, from a biological sample from an individual. Typically, the anti-amyloid antibody will be soluble in the biological sample, *e.g.*, serum, plasma, cerebrospinal fluid, lymph, urine, or other biological fluid or fraction. However, anti-amyloid antibodies can be detected in tissue biopsies as well.

In preferred methods, a biological sample (*e.g.*, blood, serum, or plasma sample) is collected or a biopsy is performed and the collected biological sample is tested *in vitro.* In certain embodiments, immunoglobulins present in the sample are enriched prior to detection. In other embodiments, immunoglobulins are not further enriched prior to detection. The tissue or fluid is then contacted with an amyloid antigen and any immune complexes that result indicate the presence of an anti-amyloid antibody in the sample. To facilitate such detection, the amyloid antigen can be radiolabeled or coupled to an effector molecule which is a detectable label, such as a fluorescent label.

Anti-amyloid antibodies and amyloid proteins and/or aggregates can be detected using standard immunoassay methods. Standard methods include, for example, radioimmunoassay, sandwich immunoassays (including ELISA), immunofluorescence assays, Western blot, affinity chromatography (affinity ligand bound to a solid phase), and in situ detection with labeled antibodies. Detectable labels suitable for such use include any composition detectable by spectroscopic, photochemical, biochemical, immunochemical, electrical, optical or chemical means. Useful labels in the present invention include magnetic beads (*e.g.*, DYNABEADS), fluorescent dyes (*e.g*., fluorescein isothiocyanate, Texas red, rhodamine green fluorescent protein, and the like), radiolabels (*e.g.*, ³H, ¹²⁵I, ³⁵S, ¹⁴C, or ³²P), enzymes (*e.g.*, horse radish peroxidase, alkaline phosphatase and others commonly used in an ELISA), and colorimetric labels such as colloidal gold or colored glass or plastic (*e.g.* polystyrene, polypropylene, latex) beads. In some embodiments, a secondary detection agent is employed to detect anti-amyloid antibody binding (*e.g.*, goat anti-human FITC). A general overview of the applicable technology can be found in Harlow & Lane, Antibodies: A Laboratory Manual (1988).

Methods currently employed for measuring anti-amyloid antibodies extracted from biological specimens suffer from artifactual gains or losses of anti-amyloid activity as a result of exposure to denaturing conditions. For example, some methods employ acidification either during enrichment of antibodies (Ig) using Protein A or G affinity chromatography or to liberate antibodies from bound antigen. Exposure to low pH causes certain mammalian immunoglobulins to wholly or partially denature. This leads either to loss of function or increased polyvalency, both of which phenomena have deleterious effects on measuring levels of specific anti-amyloid antibodies. Advantageously, methods provided herein separate Ig from plasma and dissociate weakly bound antigen molecules from the anti-amyloid antibodies without exposure to acids or other denaturing conditions.

In one embodiment, the present invention provides methods for quantifying levels of antibodies against amyloid-forming proteins in mammalian plasma and other biological fluids. Unlike existing methods, the present invention circumvents confounding effects of non-specific antibody binding, interference from plasma macromolecules and artifactual generation of binding activity by denaturation of immunoglobulin during purification.

For instance, previous assays that employed whole plasma to measure endogenous anti-amyloid activity are artifactually altered by interference from other, as yet unidentified plasma macromolecules. Advantageously, the methods provided herein eliminates the interfering activity of these unidentified plasma macromolecules in a manner that preserves the anti-amyloid antibodies of interest.

Measurements made on conventional glass, metal, or plastic substrates are affected by non-specific binding of antibodies to the amyloid protein and to the assay plates. Human plasma contains relatively large amounts of polyvalent antibodies that have low avidity for amyloid aggregates. The presence of such antibodies as well as antibodies hat bind non-specifically to assay substrates artifactually alters the measured anti-amyloid activity in various assay methods. Advantageously, the methods provided herein are able to distinguish specific from non-specific antibodies on the basis of differences in avidity of binding.

### Methods Employing a Chaotropic Wash Step

In one embodiment, the present invention provides methods for quantifying levels of antibodies against amyloid-forming proteins in mammalian plasma and other biological fluids. Unlike existing methods, the present invention circumvents confounding effects of non-specific antibody binding, interference from plasma macromolecules and artifactual generation of binding activity by denaturation of immunoglobulin during purification.

For instance, measurements made on conventional glass, metal, or plastic substrates are affected by non-specific binding of antibodies to the amyloid protein and to the assay plates. Human plasma contains relatively large amounts of polyvalent antibodies that have low avidity for amyloid aggregates. The presence of such antibodies as well as antibodies hat bind non-specifically to assay substrates artifactually alters the measured anti-amyloid activity in various assay methods. Advantageously, the methods provided herein are able to distinguish specific from non-specific antibodies on the basis of differences in avidity of binding.

Accordingly, in one aspect, the invention provides a method for detecting high avidity anti-amyloid antibodies present in a sample. In some embodiments, the method comprises the steps of (a) contacting the biological sample with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution comprising a chaotropic agent to dissociate at least one complex comprising an amyloid antigen and a low-affinity or non-specific antibody; and (c) detecting the presence or level of the remaining complexes.

In one embodiment, the biological sample is a biological fluid, for example, blood, plasma, urine, lymph, synovial fluid, *etc.* In other embodiments, the biological sample may be a tissue sample or biopsy. In a preferred embodiment, the biological sample is a blood sample or fraction thereof (*e.g.*, plasma or plasma fraction). In another preferred embodiment, the biological sample is an immunoglobulin preparation or enrichment. In a specific embodiment, the immunoglobulin enrichment is performed by thiophilic chromatography. In another specific embodiment, the immunoglobulin enrichment is performed by mixed mode ligand chromatography.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.,* dimers, trimers, etc). In yet another embodiment, the anti-amyloid antibody is specific for amyloid protein fibrils.

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e.,* dimers, trimers, *etc*). In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion.

In one embodiment, the chaotropic salt is a guanidinium salt. Non-limiting examples of guanidinium salts that may be used in conjunction with the methods provided herein include guanidinium chloride, guanidinium nitrate, and guanidinium thiocyanate.

In another embodiment, the chaotropic salt is a thiocyanate salt. Non-limiting examples of thiocyanate salts that may be used in conjunction with the methods provided herein include ammonium thiocyanate, potassium thiocyanate, sodium thiocyanate, lithium thiocyanate, calcium thiocyanate, and guanidinium thiocyanate. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

In yet another embodiment, the chaotropic salt is a perchlorate salt. Non-limiting examples of perchlorate salts that may be used in conjunction with the methods provided herein include ammonium perchlorate, sodium perchlorate, lithium perchlorate, magnesium perchlorate, and calcium perchlorate.

In yet another embodiment, the chaotropic salt is an iodide salt. Non-limiting examples of iodide salts that may be used in conjunction with the methods provided herein include ammonium iodide, potassium iodide, sodium iodide, lithium iodide, magnesium iodide, and calcium iodide.

In yet another embodiment, the chaotropic salt is a chlorate salt. Non-limiting examples of chlorate salts that may be used in conjunction with the methods provided herein include sodium chlorate, lithium chlorate, magnesium chlorate, and calcium chlorate.

The concentration of the chaotropic agent used in the wash step of methods provided herein should be selected such that it is sufficient to disrupt interactions between non-specific (*i.e.*, low avidity) anti-amyloid antibodies and amyloid antigens, yet does not disrupt interactions between specific (*i.e.* high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g.*, anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

In certain embodiments of the methods provided herein, immunoglobulins found in the biological sample (*i.e.*, blood, plasma, urine, lymph, *etc*.) are enriched prior to the step of contacting the biological sample with one or more amyloid antigens. Preferably, the immunoglobulins are enriched by a method that does not partially or completely denature the antibodies. In one embodiment, the methods provided herein for detecting high avidity anti-amyloid antibodies comprise a step of enriching immunoglobulins by a chromatographic method that does not result in denaturation of the antibodies. In one embodiment, the chromatographic method is performed with a thiophilic resin. In another embodiment, the chromatographic method is performed with mixed mode ligand chemistry (Upfront Chromatography A/S). In other embodiments, the methods provided herein for detecting high avidity anti-amyloid antibodies comprise a step of enriching immunoglobulins by a non-chromatographic method that does not result in denaturation of the antibodies.

### Methods Employing a Thiophilic Enrichment Step

In one embodiment, the present invention provides methods for quantifying levels of antibodies against amyloid-forming proteins in mammalian plasma and other biological fluids. Unlike existing methods, the present invention circumvents confounding effects of non-specific antibody binding, interference from plasma macromolecules and artifactual generation of binding activity by denaturation of immunoglobulin during purification.

For instance, previous assays that employed whole plasma to measure endogenous anti-amyloid activity are artifactually altered by interference from other, as yet unidentified plasma macromolecules. Advantageously, the methods provided herein eliminates the interfering activity of these unidentified plasma macromolecules in a manner that preserves the anti-amyloid antibodies of interest.

Methods currently employed for measuring anti-amyloid antibodies extracted from biological specimens suffer from artifactual gains or losses of anti-amyloid activity as a result of exposure to denaturing conditions. For example, some methods employ acidification either during enrichment of antibodies (Ig) using Protein A or G affinity chromatography or to liberate antibodies from bound antigen. Exposure to low pH causes certain mammalian immunoglobulins to wholly or partially denature. This leads either to loss of function or increased polyvalency, both of which phenomena have deleterious effects on measuring levels of specific anti-amyloid antibodies. Advantageously, methods provided herein separate Ig from plasma and dissociate weakly bound antigen molecules from the anti-amyloid antibodies without exposure to acids or other denaturing conditions.

Accordingly, in one aspect, the invention provides a method detecting a high avidity anti-amyloid antibody present in a biological sample, the method comprising the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; and (d) detecting the presence or level of the complex.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.*, dimers, trimers, *etc*). In yet another embodiment, the anti-amyloid antibody is specific for amyloid protein fibrils.

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e.,* dimers, trimers, *etc*). In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

In certain embodiments of the methods provided herein, the complex comprising the amyloid antigen and the anti-amyloid antibody is washed with a solution containing a chaotropic salt prior to detecting the presence or level of said complex in order to dissociate interactions between non-specific antibodies (*i.e.*, low avidity antibodies) and the amyloid antigen. The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion. In one embodiment, the chaotropic agent is selected from urea, thiourea, a guanidinium salt, a thiocyanate salt, a perchlorate salt, an iodide salt, and a chlorate salt. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

The concentration of the chaotropic agent used in the wash step of methods provided herein should be selected such that it is sufficient to disrupt interactions between non-specific (*i.e.*, low avidity) anti-amyloid antibodies and amyloid antigens, yet does not disrupt interactions between specific (*i.e.* high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g.*, anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

### Diagnostic and Prognostic Methods

In certain aspects the methods provided herein for detecting anti-amyloid antibodies can be used to for diagnosis and prognosis of individuals at risk of, or suffering from, amyloid related diseases as described above. For example, high levels of amyloid, in particular, Aβ, in serum and certain tissues can indicate an increased likelihood of developing the amyloid plaques associated with Alzheimer's Disease. High levels of amyloid-specific auto-antibodies also indicate that an individual is at risk for AD, or a worsening of AD-related symptoms.

### Methods for Diagnosing and/or Selecting a Suitable Treatment

Thus, in some embodiments, the invention provides methods for diagnosis and/or selecting a course of treatment for a subject having or suspected of having an amyloid-related disorder. In some embodiments, the methods comprise detection and/or measurement of auto-antibodies specific for an amyloid protein. Methods for detecting endogenous antibodies having high avidity for amyloid proteins are outlined herein.

In one embodiment, the methods for detecting anti-amyloid antibodies can comprise obtaining an antibody-containing sample from a subject, conveniently a blood or plasma sample. The sample can be further separated, *e.g.*, into plasma and cellular matter, to isolate the antibody-containing fraction, although this step is not necessary. The sample can also be exposed to size filtration and/or chromatography methods. In some embodiments, the sample is exposed to thiophilic chromatography to remove non-immunoglobulin proteins from the sample. The sample, or eluent, can then be contacted with an amyloid antigen in a system designed to detect an interaction between the antibody and the antigen, for example an antigen conjugated to a convenient matrix, *e.g.*, an ELISA plate or chromatography matrix. The amyloid antigen can be in any form, either heterogenous amyloid, or largely homogenous amyloid monomer, oligomer, or fibril. After sufficient incubation to allow formation of amyloid-antibody complexes, the amyloid-antibody complexes are exposed to a wash comprising a chaotropic agent, to remove low-affinity and non-specific antibodies. After the chaotropic wash, the presence or level of antibody complexed with amyloid antigen is detected. The antibody can be eluted from the antigen prior to detection if desired, or competed away with a labeled amyloid antigen for ease of detection. The antibody can be detected as described above.

In some embodiments, at least one control is run alongside the sample, and compared for amount of antibody and/or level of binding. In other embodiments, the result of the assay may be compared to a control level previously established for the system of interest. For example, a positive control can include the same biological sample obtained from an individual or group of individuals that is known to have an amyloid-related disease. Another example of a suitable positive control is a known anti-amyloid monoclonal antibody, *e.g.*, as a high affinity and/or avidity comparison. An exemplary negative control can include the same biological sample obtained from an individual or group of individuals that have low risk of developing the amyloid related disease. Another example of a suitable negative control is a known antibody specific for a non-amyloid antigen or having low avidity for the amyloid antigen.

Using such methods, and correlating a relatively high level of anti-amyloid antibodies with an increased likelihood that the subject has or is at high risk of developing an amyloid-related disorder, one of skill can diagnose an amyloid-related disease in the subject.

The above methods can be applied to selecting a patient group for an amyloid-related disease therapy. For example, the level of anti-amyloid antibodies can be determined in a plurality of individuals. As explained above, those individuals determined to have relatively high levels of anti-amyloid antibodies can be selected for treatment. In some embodiment, the level of anti-amyloid antibodies is detected periodically over a course of treatment. In some embodiments, the treatment comprises administration of IVIG.

### Methods Employing a Chaotropic Wash Step

In one aspect, the present invention provides methods for identifying a subject who is a candidate for treatment of an amyloid-related disease or condition, comprising quantifying levels of antibodies against amyloid-forming proteins in mammalian plasma and other biological fluids, wherein a chaotropic wash step is employed to reduce the signal associated with non-specific and low-avidity anti-amyloid antigen binding. In a preferred embodiment, the treatment comprises administration of an immunoglobulin preparation. In a specific embodiment, the amyloid-related disease is Alzheimer's disease.

In a related aspect, the present invention provides methods for diagnosing a disease associated with an amyloid protein, comprising quantifying levels of antibodies against amyloid-forming proteins in mammalian plasma and other biological fluids, wherein a chaotropic wash step is employed to reduce the signal associated with non-specific and low-avidity anti-amyloid antigen binding. In a preferred embodiment, the method is for diagnosing Alzheimer's disease.

Unlike existing methods, the present invention circumvents confounding effects of non-specific antibody binding, interference from plasma macromolecules and artifactual generation of binding activity by denaturation of immunoglobulin during purification.

For instance, measurements made on conventional glass, metal, or plastic substrates are affected by non-specific binding of antibodies to the amyloid protein and to the assay plates. Human plasma contains relatively large amounts of polyvalent antibodies that have low avidity for amyloid aggregates. The presence of such antibodies as well as antibodies hat bind non-specifically to assay substrates artifactually alters the measured anti-amyloid activity in various assay methods. Advantageously, the methods provided herein are able to distinguish specific from non-specific antibodies on the basis of differences in avidity of binding.

Accordingly, in a specific embodiment, the invention provides a method for identifying a subject who is a candidate for treatment of an amyloid-related disease or condition. In some embodiments, the method comprises the steps of (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific anti-amyloid antibody; (c) detecting the level of the remaining complexes; and (d) determining whether the subject would benefit from a treatment for an amyloid-related disease or condition by comparing the level of the anti-amyloid antibody to a control level. In a preferred embodiment, the treatment comprises the administration of an immunoglobulin preparation.

In some embodiments of the methods for identifying a subject who is a candidate for treatment, the control is from an individual or group of individuals that do not have an amyloid-related disease, such that an increased level of the remaining complex relative to control is indicative of a candidate for treatment. In some embodiments, the control is from an individual or group of individuals that do have an amyloid-related disease, such that a similar level of the remaining complex relative to control is indicative of a candidate for treatment. In yet another embodiment, the control is from an individual or group of individuals that have been diagnosed with an amyloid-related disease and who have responded favorably to a particular course of treatment. In another embodiment, the control is from an individual or group of individuals that have been diagnosed with an amyloid-related disease and who have not responded favorably to a particular course of treatment. One of skill will understand how to select at least one appropriate control and interpret the results accordingly.

In certain embodiments, the methods for identifying a subject who is a candidate for treatment further comprise a step of assigning a course of treatment or administering a treatment to the subject. Generally, this course of treatment will be assigned when the level of anti-amyloid antibodies in the biological sample is above a control threshold (*e.g.*, a threshold indicating that the subject likely has an amyloid-related condition or is likely to respond to a particular treatment), or more closely resembles a positive control (*e.g.*, a control level from a group or individual having an amyloid-related condition or a control level from a group or individual who has responded favorably to a particular treatment) than to a negative control (*e.g.*, a control level from a group or individual not having an amyloid-related condition or a control level from a group or individual who has not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g.*, Memantine), and a psychiatric drugs (*e.g.*, antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises identifying a subject who is a candidate for treatment of Alzheimer's disease.

In a related embodiment, the present invention provides a method for diagnosing a disease associated with an amyloid protein in a subject. In some embodiments, the method comprises the steps of (a) contacting a biological sample from the subject with a plurality of amyloid antigens under suitable conditions to form:
(i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) diagnosing the subject by comparing the level of the anti-amyloid antibody to a control level.

In some embodiments of the methods for diagnosing a disease associated with an amyloid protein in a subject, the control is from an individual or group of individuals that do not have an amyloid-related disease, such that an increased level of the remaining complex relative to control is indicative of a high likelihood that the subject has an amyloid-related condition. In some embodiments, the control is from an individual or group of individuals that do have an amyloid-related disease, such that a similar level of the remaining complex relative to control is indicative of a high likelihood that the subject has an amyloid-related condition. One of skill will understand how to select at least one appropriate control and interpret the results accordingly.

In certain embodiments, the methods for diagnosing a disease associated with an amyloid protein in a subject further comprise a step of assigning a course of treatment or administering a treatment to the subject. Generally, this course of treatment will be assigned when the level of anti-amyloid antibodies in the biological sample is above a control threshold (*e.g.*, a threshold indicating that the subject likely has an amyloid-related condition or is likely to respond to a particular treatment), or more closely resembles a positive control (*e.g.*, a control level from a group or individual having an amyloid-related condition or a control level from a group or individual who has responded favorably to a particular treatment) than to a negative control (*e.g.*, a control level from a group or individual not having an amyloid-related condition or a control level from a group or individual who has not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g.*, Memantine), and a psychiatric drugs (*e.g.*, antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In one embodiment of the methods for identifying a subject who is a candidate for treatment or method for diagnosing a disease associated with an amyloid protein in a subject, the biological sample is a biological fluid, for example, blood, plasma, urine, lymph, synovial fluid, *etc.* In other embodiments, the biological sample may be a tissue sample or biopsy. In a preferred embodiment, the biological sample is a blood sample or fraction thereof (*e.g.*, plasma or plasma fraction). In another preferred embodiment, the biological sample is an immunoglobulin preparation or enrichment. In a specific embodiment, the immunoglobulin enrichment is performed by thiophilic chromatography. In another specific embodiment, the immunoglobulin enrichment is performed by mixed mode ligand chromatography.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.*, dimers, trimers, *etc*). In yet another embodiment, the anti-amyloid antibody is specific for amyloid protein fibrils.

In one embodiment, the disease or condition associated with an amyloid protein is selected from the group consisting of Alzheimer's disease, Type 2 diabetes mellitus, Parkinson's disease, Transmissible spongiform encephalopathy, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), and systemic AL amyloidosis. In a preferred embodiment, the disease or condition is Alzheimer's disease. In a particularly preferred embodiment, the disease or condition is Alzheimer's disease and the anti-amyloid antibodies being detected are anti-Beta amyloid (Aβ; Abeta) antibodies.

In certain embodiments of the methods provided herein, detection of the presence or level of a particular amyloid protein is useful to diagnose a particular disease or condition, or to select a candidate for the treatment of a particular disease or condition. Non-limiting examples of amyloid-amyloid disease combinations that are well suited for use in the methods provided herein can be found in Table 1. In certain embodiments, the detection of the presence or level of a high-avidity antibody specific for the amyloid protein listed in Table 1 will be diagnostic of the corresponding disease listed.

**Table 1. Non-limiting examples of amyloid proteins associated with specific diseases.**

| **Disease** | **Amyloid Protein** | **GenBank Accession** | **UniProt Accession** |
|---|---|---|---|
| Alzheimer's disease | Beta amyloid (Aβ; Abeta) | NP_000475 | P05067 |
| Type 2 diabetes mellitus | Islet amyloid polypeptide (IAPP; Amylin) | NP_000406 | P10997 |
| Parkinson's disease | Alpha-synuclein (SNCA) | NP_000336 | P37840 |
| Transmissible spongiform encephalopathy (*e.g.*, Creutzfeldt-Jakob disease) | Major Prion Protein (PrP) | NP_000302 | P04156 |
| Huntington's Disease | Huntingtin (HD) | NP_002102 | P42858 |
| Medullary carcinoma of the thyroid | Calcitonin (CCP) | NP_001029124 | P01258 |
| Cardiac arrhythmias, Isolated atrial amyloidosis | Atrial natriuretic factor (ANF) | NP_006163 | P01160 |
| Atherosclerosis | Apolipoprotein AI (Apo-A1) | NP_000030 | P02647 |
| Rheumatoid arthritis | Serum amyloid A protein (SAA) | NP_954630 | P02735 |
| Aortic medial amyloid | Medin amyloid (fragment of Milk fat globule-EGF factor 8 protein; MFG-E8) | NP_005919 | Q08431 |
| Prolactinomas | Prolactin (PRL) | NP_000939 | P01236 |
| Familial amyloid polyneuropathy | Transthyretin (ATTR) | NP_000362 | P02766 |
| Hereditary non-neuropathic systemic amyloidosis | Lysozyme C (1,4-beta-N-acetylmuramidase C) | NP_000230 | P61626 |
| Dialysis related amyloidosis | Beta 2 microglobulin (β2M) | NP_004039 | P61769 |
| Finnish amyloidosis | Gelsolin (AGEL) | NP_000168 | P06396 |
| Lattice corneal dystrophy | Transforming growth factor-beta-induced protein ig-h3 (Beta ig-h3; Keratoepithelin) | NP_000349 | Q15582 |
| Cerebral amyloid angiopathy | Beta amyloid (Aβ; Abeta) | NP_000475 | P05067 |
| Cerebral amyloid angiopathy (Icelandic type) | Cystatin C (CST3) | NP_000090 | P01034 |
| systemic AL amyloidosis | Immunoglobulin light chain (AL) | | |

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e.*, dimers, trimers, *etc*). In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion.

In one embodiment, the chaotropic salt is a guanidinium salt. Non-limiting examples of guanidinium salts that may be used in conjunction with the methods provided herein include guanidinium chloride, guanidinium nitrate, and guanidinium thiocyanate.

In another embodiment, the chaotropic salt is a thiocyanate salt. Non-limiting examples of thiocyanate salts that may be used in conjunction with the methods provided herein include ammonium thiocyanate, potassium thiocyanate, sodium thiocyanate, lithium thiocyanate, calcium thiocyanate, and guanidinium thiocyanate. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

In yet another embodiment, the chaotropic salt is a perchlorate salt. Non-limiting examples of perchlorate salts that may be used in conjunction with the methods provided herein include ammonium perchlorate, sodium perchlorate, lithium perchlorate, magnesium perchlorate, and calcium perchlorate.

In yet another embodiment, the chaotropic salt is an iodide salt. Non-limiting examples of iodide salts that may be used in conjunction with the methods provided herein include ammonium iodide, potassium iodide, sodium iodide, lithium iodide, magnesium iodide, and calcium iodide.

In yet another embodiment, the chaotropic salt is a chlorate salt. Non-limiting examples of chlorate salts that may be used in conjunction with the methods provided herein include sodium chlorate, lithium chlorate, magnesium chlorate, and calcium chlorate.

The concentration of the chaotropic agent used in the wash step of methods provided herein should be selected such that it is sufficient to disrupt interactions between non-specific (*i.e.,* low avidity) anti-amyloid antibodies and amyloid antigens, yet does not disrupt interactions between specific (*i.e.* high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g.*, anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

In certain embodiments of the methods provided herein, immunoglobulins found in the biological sample (*i.e.,* blood, plasma, urine, lymph, *etc*.) are enriched prior to the step of contacting the biological sample with one or more amyloid antigens. Preferably, the immunoglobulins are enriched by a method that does not partially or completely denature the antibodies. In one embodiment, the methods provided herein for detecting high avidity anti-amyloid antibodies comprise a step of enriching immunoglobulins by a chromatographic method that does not result in denaturation of the antibodies. In one embodiment, the chromatographic method is performed with a thiophilic resin. In another embodiment, the chromatographic method is performed with mixed mode ligand chemistry (Upfront Chromatography A/S). In other embodiments, the methods provided herein for detecting high avidity anti-amyloid antibodies comprise a step of enriching immunoglobulins by a non-chromatographic method that does not result in denaturation of the antibodies.

### Methods Employing a Thiophilic Enrichment Step

In one aspect, the present invention provides methods for identifying a subject who is a candidate for treatment, comprising administration of an immunoglobulin preparation comprising quantifying levels of antibodies against amyloid-forming proteins in mammalian plasma and other biological fluids, wherein a thiophilic enrichment step is employed to reduce the signal associated with non-specific antibody binding, interference from plasma macromolecules and artifactual generation of binding activity by denaturation of immunoglobulin during purification.

In a related aspect, the present invention provides methods for diagnosing a disease associated with an amyloid protein, comprising administration of an immunoglobulin preparation comprising quantifying levels of antibodies against amyloid-forming proteins in mammalian plasma and other biological fluids, wherein a thiophilic enrichment step is employed to reduce the signal associated with non-specific antibody binding, interference from plasma macromolecules and artifactual generation of binding activity by denaturation of immunoglobulin during purification.

Unlike existing methods, the present invention circumvents confounding effects of non-specific antibody binding, interference from plasma macromolecules and artifactual generation of binding activity by denaturation of immunoglobulin during purification.

For instance, previous assays that employed whole plasma to measure endogenous anti-amyloid activity are artifactually altered by interference from other, as yet unidentified plasma macromolecules. Advantageously, the methods provided herein eliminates the interfering activity of these unidentified plasma macromolecules in a manner that preserves the anti-amyloid antibodies of interest.

Methods currently employed for measuring anti-amyloid antibodies extracted from biological specimens suffer from artifactual gains or losses of anti-amyloid activity as a result of exposure to denaturing conditions. For example, some methods employ acidification either during enrichment of antibodies (Ig) using Protein A or G affinity chromatography or to liberate antibodies from bound antigen. Exposure to low pH causes certain mammalian immunoglobulins to wholly or partially denature. This leads either to loss of function or increased polyvalency, both of which phenomena have deleterious effects on measuring levels of specific anti-amyloid antibodies. Advantageously, methods provided herein separate Ig from plasma and dissociate weakly bound antigen molecules from the anti-amyloid antibodies without exposure to acids or other denaturing conditions.

Accordingly, in a specific embodiment, the invention provides a method for identifying a subject who is a candidate for treatment of an amyloid-related disease or condition. In some embodiments, the method comprises the steps of (a) contacting the biological sample with a thiophilic resin to bind an anti-amyloid antibody present in the sample; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) determining whether the subject would benefit from a treatment comprising administration of an immunoglobulin preparation by comparing the level of the anti-amyloid antibody to a control level. In a preferred embodiment, the treatment comprises the administration of an immunoglobulin preparation.

In some embodiments of the methods for identifying a subject who is a candidate for treatment, the control is from an individual or group of individuals that do not have an amyloid-related disease, such that an increased level of the remaining complex relative to control is indicative of a candidate for treatment. In some embodiments, the control is from an individual or group of individuals that do have an amyloid-related disease, such that a similar level of the remaining complex relative to control is indicative of a candidate for treatment. In yet another embodiment, the control is from an individual or group of individuals that have been diagnosed with an amyloid-related disease and who have responded favorably to a particular course of treatment. In another embodiment, the control is from an individual or group of individuals that have been diagnosed with an amyloid-related disease and who have not responded favorably to a particular course of treatment. One of skill will understand how to select at least one appropriate control and interpret the results accordingly.

In certain embodiments, the methods for identifying a subject who is a candidate for treatment further comprise a step of assigning a course of treatment or administering a treatment to the subject comprising administration of an immunoglobulin preparation. Generally, this course of treatment will be assigned when the level of anti-amyloid antibodies in the biological sample is above a control threshold (*e.g.*, a threshold indicating that the subject likely has an amyloid-related condition or is likely to respond to a particular treatment), or more closely resembles a positive control (*e.g.*, a control level from a group or individual having an amyloid-related condition or a control level from a group or individual who has responded favorably to a particular treatment) than to a negative control (*e.g.*, a control level from a group or individual not having an amyloid-related condition or a control level from a group or individual who has not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g.*, Memantine), and a psychiatric drugs (*e.g.*, antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises identifying a subject who is a candidate for treatment of Alzheimer's disease.

In a related embodiment, the present invention provides a method for diagnosing a disease associated with an amyloid protein in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) diagnosing the subject by comparing the level of the anti-amyloid antibody to a control level.

In some embodiments of the methods for diagnosing a disease associated with an amyloid protein in a subject, the control is from an individual or group of individuals that do not have an amyloid-related disease, such that an increased level of the remaining complex relative to control is indicative of a high likelihood that the subject has an amyloid-related condition. In some embodiments, the control is from an individual or group of individuals that do have an amyloid-related disease, such that a similar level of the remaining complex relative to control is indicative of a high likelihood that the subject has an amyloid-related condition. One of skill will understand how to select at least one appropriate control and interpret the results accordingly.

In certain embodiments, the methods for diagnosing a disease associated with an amyloid protein in a subject further comprise a step of assigning a course of treatment or administering a treatment to the subject. Generally, this course of treatment will be assigned when the level of anti-amyloid antibodies in the biological sample is above a control threshold (*e.g.*, a threshold indicating that the subject likely has an amyloid-related condition or is likely to respond to a particular treatment), or more closely resembles a positive control (*e.g.*, a control level from a group or individual having an amyloid-related condition or a control level from a group or individual who has responded favorably to a particular treatment) than to a negative control (*e.g.*, a control level from a group or individual not having an amyloid-related condition or a control level from a group or individual who has not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g.*, Memantine), and a psychiatric drugs (*e.g.*, antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In one embodiment of the methods for identifying a subject who is a candidate for treatment or method for diagnosing a disease associated with an amyloid protein in a subject, the biological sample is a biological fluid, for example, blood, plasma, urine, lymph, synovial fluid, *etc.* In other embodiments, the biological sample may be a tissue sample or biopsy. In a preferred embodiment, the biological sample is a blood sample or fraction thereof (*e.g.*, plasma or plasma fraction). In another preferred embodiment, the biological sample is an immunoglobulin preparation or enrichment. In a specific embodiment, the immunoglobulin enrichment is performed by thiophilic chromatography. In another specific embodiment, the immunoglobulin enrichment is performed by mixed mode ligand chromatography.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.,* dimers, trimers, *etc*). In yet another embodiment, the anti-amyloid antibody is specific for amyloid protein fibrils.

In one embodiment, the disease or condition associated with an amyloid protein is selected from the group consisting of Alzheimer's disease, Type 2 diabetes mellitus, Parkinson's disease, Transmissible spongiform encephalopathy, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), and systemic AL amyloidosis. In a preferred embodiment, the disease or condition is Alzheimer's disease. In a particularly preferred embodiment, the disease or condition is Alzheimer's disease and the anti-amyloid antibodies being detected are anti-Beta amyloid (Aβ; Abeta) antibodies.

In certain embodiments of the methods provided herein, detection of the presence or level of a particular amyloid protein is useful to diagnose a particular disease or condition, or to select a candidate for the treatment of a particular disease or condition. Non-limiting examples of amyloid-amyloid disease combinations that are well suited for use in the methods provided herein can be found in Table 1. In certain embodiments, the detection of the presence or level of a high-avidity antibody specific for the amyloid protein listed in Table 1 will be diagnostic of the corresponding disease listed.

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e.,* dimers, trimers, *etc*). In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

In certain embodiments of the methods provided herein, the complex comprising the amyloid antigen and the anti-amyloid antibody is washed with a solution containing a chaotropic salt prior to detecting the presence or level of said complex in order to dissociate interactions between non-specific antibodies *(i.e.,* low avidity antibodies) and the amyloid antigen. The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion. In one embodiment, the chaotropic agent is selected from urea, thiourea, a guanidinium salt, a thiocyanate salt, a perchlorate salt, an iodide salt, and a chlorate salt. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

The concentration of the chaotropic agent used in the wash step of methods provided herein should be selected such that it is sufficient to disrupt interactions between non-specific (*i.e.,* low avidity) anti-amyloid antibodies and amyloid antigens, yet does not disrupt interactions between specific (*i.e*. high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g*., anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

### Methods for Providing a Prognosis

In some embodiments, the detection of anti-amyloid antibody levels can provide a prognosis for a subject. The subject can be untreated or undiagnosed, or currently undergoing treatment or taking preventative measures against an amyloid-related disease. Thus, the level of anti-amyloid antibodies can be periodically detected to monitor the subject over time. In a specific embodiment, the detection of high levels of anti-amyloid antibodies can provide a prognosis for the subject.

In some embodiments, the methods of detecting anti-amyloid antibodies can be used to decide on a therapeutic regime. In cases where there appears to be relatively low risk of developing an amyloid-related disease, the subject can be advised to take preventative measures. In some cases, however, where the risk of developing an amyloid related disease is determined to be higher, a treatment can be prescribed. The treatment can comprise administration of an immunoglobulin preparation, *e.g*., a pooled immunoglobulin preparation such as IVIG or other forms of immunotherapy.

Methods to detect amyloid are outlined above. For example, the method can include obtaining from a subject a biological sample, contacting the sample with an amyloid protein or antigen thereof, detecting the presence or absence of anti-amyloid antibody binding, and diagnosing an increased likelihood of developing an amyloid-related disorder based on levels of high avidity anti-amyloid antibodies in the sample as compared to a control. In some embodiments, the method further comprises selecting a course of treatment appropriate to the subject. In some embodiments, the treatment comprises administering IVIG as described herein to the subject.

### Methods Employing a Chaotropic Wash Step

In one aspect, the present invention provides methods for providing a prognosis for the progression of a disease or condition associated with an amyloid protein, comprising quantifying levels of antibodies against amyloid-forming proteins in mammalian plasma and other biological fluids, wherein a chaotropic wash step is employed to reduce the signal associated with non-specific and low-avidity anti-amyloid antigen binding.

In a related aspect, the present invention provides methods for providing a prognosis for treatment of a disease or condition associated with an amyloid protein, comprising quantifying levels of antibodies against amyloid-forming proteins in mammalian plasma and other biological fluids, wherein a chaotropic wash step is employed to reduce the signal associated with non-specific and low-avidity anti-amyloid antigen binding.

Unlike existing methods, the present invention circumvents confounding effects of non-specific antibody binding, interference from plasma macromolecules and artifactual generation of binding activity by denaturation of immunoglobulin during purification.

For instance, Measurements made on conventional glass, metal, or plastic substrates are affected by non-specific binding of antibodies to the amyloid protein and to the assay plates. Human plasma contains relatively large amounts of polyvalent antibodies that have low avidity for amyloid aggregates. The presence of such antibodies as well as antibodies hat bind non-specifically to assay substrates artifactually alters the measured anti-amyloid activity in various assay methods. Advantageously, the methods provided herein are able to distinguish specific from non-specific antibodies on the basis of differences in avidity of binding.

Accordingly, in a specific embodiment, the invention provides a method for providing a prognosis for the progression of a disease associated with an amyloid protein in a subject. In some embodiments, the method comprises the steps of (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) providing a prognosis for the progression of the disease by comparing the level of the anti-amyloid antibody to a control level.

In some embodiments of the methods for providing a prognosis for the progression of a disease associated with an amyloid protein, the control is from an individual or group of individuals that experienced progression of the disease, such that a similar or increased level of the remaining complex relative to control is indicative of a high likelihood or progression of the disease. In some embodiments, the control is from an individual or group of individuals that did not experience progression of the disease, such that a similar level of the remaining complex relative to control is indicative of a low likelihood of progression of the disease. In yet another embodiment, the control is from the same patient taken at an earlier time, such that an increased level of the remaining complex relative to control is indicative of a high likelihood or progression of the disease. In certain embodiments, the previous sample may have been taken about 1 month prior, or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 month prior, or 4, 5, 6, 7, 8, 9, 10 or more years prior. One of skill will understand how to select at least one appropriate control and interpret the results accordingly.

In certain embodiments, the methods for providing a prognosis for the progression of a disease associated with an amyloid protein further comprise a step of assigning a course of treatment or administering a treatment to the subject Generally, this course of treatment will be assigned when the level of anti-amyloid antibodies in the biological sample is above a control threshold (*e.g*., a threshold indicating a high likelihood or progression of the disease), or more closely resembles a positive control (*e.g*., a control level from a group or individual having experienced progression of the disease) than to a negative control (*e.g*., a control level from a group or individual not having experienced progression of the disease). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g*., Memantine), and a psychiatric drugs (*e.g*., antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In a related embodiment, the present invention provides a method for providing a prognosis for treatment of a disease associated with an amyloid protein, for example a known therapy or preventative strategy for AD. In some embodiments, the treatment can comprise administration of an immunoglobulin preparation such as IVIG in a subject. In some embodiments, the method comprises the steps of (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; (b) washing the amyloid antigen complexes formed in step (a) with a solution containing a chaotropic agent to dissociate at least one complex comprising an amyloid antigen and a low-affinity or non-specific antibody; (c) detecting the level of the remaining complexes; and (d) providing a prognosis for treatment of the disease by comparing the level of the anti-amyloid antibody to a control level.

In some embodiments of the methods for providing a prognosis for treatment of a disease associated with an amyloid protein, the control is from an individual or group of individuals that responded favorably to a particular treatment, such that a similar level of the remaining complex relative to control is indicative of a good prognosis for the treatment of the disease. In some embodiments, the control is from an individual or group of individuals that did not responded favorably to a particular treatment, such that a similar level of the remaining complex relative to control is indicative of a poor prognosis for the treatment of the disease. One of skill will understand how to select at least one appropriate control and interpret the results accordingly.

In certain embodiments, the methods for providing a prognosis for treatment of a disease associated with an amyloid protein further comprise a step of assigning a course of treatment or administering a treatment to the subject. Generally, this course of treatment will be assigned when the level of anti-amyloid antibodies in the biological sample more closely resembles a positive control (*e.g*., a control level from a group or individual that responded favorably to a particular treatment) than to a negative control (*e.g.*, a control level from a group or individual that did not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g*., Memantine), and a psychiatric drugs (*e.g*., antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In one embodiment of the methods for providing a prognosis for the progression of a disease associated with an amyloid protein or for providing a prognosis for treatment of a disease associated with an amyloid protein, the biological sample is a biological fluid, for example, blood, plasma, urine, lymph, synovial fluid, *etc.* In other embodiments, the biological sample may be a tissue sample or biopsy. In a preferred embodiment, the biological sample is a blood sample or fraction thereof (*e.g*., plasma or plasma fraction). In another preferred embodiment, the biological sample is an immunoglobulin preparation or enrichment. In a specific embodiment, the immunoglobulin enrichment is performed by thiophilic chromatography. In another specific embodiment, the immunoglobulin enrichment is performed by mixed mode ligand chromatography.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.,* dimers, trimers, *etc*)*.* In yet another embodiment, the anti-amyloid antibody is specific for amyloid protein fibrils.

In one embodiment, the disease or condition associated with an amyloid protein is selected from the group consisting of Alzheimer's disease, Type 2 diabetes mellitus, Parkinson's disease, Transmissible spongiform encephalopathy, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), and systemic AL amyloidosis. In a preferred embodiment, the disease or condition is Alzheimer's disease. In a particularly preferred embodiment, the disease or condition is Alzheimer's disease and the anti-amyloid antibodies being detected are anti-Beta amyloid (Aβ; Abeta) antibodies.

In certain embodiments of the methods provided herein, detection of the presence or level of a particular amyloid protein is useful to provide a prognosis for the progression of a disease or provide a prognosis for the treatment of a disease associated with an amyloid protein. Non-limiting examples of amyloid-amyloid disease combinations that are well suited for use in the methods provided herein can be found in Table 1. In certain embodiments, the detection of the presence or level of a high-avidity antibody specific for the amyloid protein listed in Table 1 will be diagnostic of the corresponding disease listed.

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e*., dimers, trimers, *etc*)*.* In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion.

In one embodiment, the chaotropic salt is a guanidinium salt. Non-limiting examples of guanidinium salts that may be used in conjunction with the methods provided herein include guanidinium chloride, guanidinium nitrate, and guanidinium thiocyanate.

In another embodiment, the chaotropic salt is a thiocyanate salt. Non-limiting examples of thiocyanate salts that may be used in conjunction with the methods provided herein include ammonium thiocyanate, potassium thiocyanate, sodium thiocyanate, lithium thiocyanate, calcium thiocyanate, and guanidinium thiocyanate. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

In yet another embodiment, the chaotropic salt is a perchlorate salt. Non-limiting examples of perchlorate salts that may be used in conjunction with the methods provided herein include ammonium perchlorate, sodium perchlorate, lithium perchlorate, magnesium perchlorate, and calcium perchlorate.

In yet another embodiment, the chaotropic salt is an iodide salt. Non-limiting examples of iodide salts that may be used in conjunction with the methods provided herein include ammonium iodide, potassium iodide, sodium iodide, lithium iodide, magnesium iodide, and calcium iodide.

In yet another embodiment, the chaotropic salt is a chlorate salt. Non-limiting examples of chlorate salts that may be used in conjunction with the methods provided herein include sodium chlorate, lithium chlorate, magnesium chlorate, and calcium chlorate.

The concentration of the chaotropic agent used in the wash step of methods provided herein should be selected such that it is sufficient to disrupt interactions between non-specific (*i.e.,* low avidity) anti-amyloid antibodies and amyloid antigens, yet does not disrupt interactions between specific (*i.e*. high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g*., anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

In certain embodiments of the methods provided herein, immunoglobulins found in the biological sample (*i.e.,* blood, plasma, urine, lymph, *etc.*) are enriched prior to the step of contacting the biological sample with one or more amyloid antigens. Preferably, the immunoglobulins are enriched by a method that does not partially or completely denature the antibodies. In one embodiment, the methods provided herein for detecting high avidity anti-amyloid antibodies comprise a step of enriching immunoglobulins by a chromatographic method that does not result in denaturation of the antibodies. In one embodiment, the chromatographic method is performed with a thiophilic resin. In another embodiment, the chromatographic method is performed with mixed mode ligand chemistry (Upfront Chromatography A/S). In other embodiments, the methods provided herein for detecting high avidity anti-amyloid antibodies comprise a step of enriching immunoglobulins by a non-chromatographic method that does not result in denaturation of the antibodies.

### Methods Employing a Thiophilic Enrichment Step

In one aspect, the present invention provides methods for providing a prognosis for the progression of a disease or condition associated with an amyloid protein, comprising quantifying levels of antibodies against amyloid-forming proteins in mammalian plasma and other biological fluids, wherein a thiophilic enrichment step is employed to reduce the signal associated with non-specific antibody binding, interference from plasma macromolecules and artifactual generation of binding activity by denaturation of immunoglobulin during purification.

In a related aspect, the present invention provides methods for providing a prognosis for treatment of a disease or condition associated with an amyloid protein, comprising quantifying levels of antibodies against amyloid-forming proteins in mammalian plasma and other biological fluids, wherein a thiophilic enrichment step is employed to reduce the signal associated with non-specific antibody binding, interference from plasma macromolecules and artifactual generation of binding activity by denaturation of immunoglobulin during purification.

Unlike existing methods, the present invention circumvents confounding effects of non-specific antibody binding, interference from plasma macromolecules and artifactual generation of binding activity by denaturation of immunoglobulin during purification.

For instance, previous assays that employed whole plasma to measure endogenous anti-amyloid activity are artifactually altered by interference from other, as yet unidentified plasma macromolecules. Advantageously, the methods provided herein eliminates the interfering activity of these unidentified plasma macromolecules in a manner that preserves the anti-amyloid antibodies of interest.

Methods currently employed for measuring anti-amyloid antibodies extracted from biological specimens suffer from artifactual gains or losses of anti-amyloid activity as a result of exposure to denaturing conditions. For example, some methods employ acidification either during enrichment of antibodies (Ig) using Protein A or G affinity chromatography or to liberate antibodies from bound antigen. Exposure to low pH causes certain mammalian immunoglobulins to wholly or partially denature. This leads either to loss of function or increased polyvalency, both of which phenomena have deleterious effects on measuring levels of specific anti-amyloid antibodies. Advantageously, methods provided herein separate Ig from plasma and dissociate weakly bound antigen molecules from the anti-amyloid antibodies without exposure to acids or other denaturing conditions.

Accordingly, in a specific embodiment, the invention provides a method for providing a prognosis for the progression of a disease associated with an amyloid protein in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) providing a prognosis for the progression of the disease by comparing the level of the anti-amyloid antibody to a control level.

In some embodiments of the methods for providing a prognosis for the progression of a disease associated with an amyloid protein, the control is from an individual or group of individuals that experienced progression of the disease, such that a similar or increased level of the remaining complex relative to control is indicative of a high likelihood or progression of the disease. In some embodiments, the control is from an individual or group of individuals that did not experience progression of the disease, such that a similar level of the remaining complex relative to control is indicative of a low likelihood of progression of the disease. In yet another embodiment, the control is from the same patient taken at an earlier time, such that an increased level of the remaining complex relative to control is indicative of a high likelihood or progression of the disease. In certain embodiments, the previous sample may have been taken about 1 month prior, or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 month prior, or 4, 5, 6, 7, 8, 9,10 or more years prior. One of skill will understand how to select at least one appropriate control and interpret the results accordingly.

In certain embodiments, the methods for providing a prognosis for the progression of a disease associated with an amyloid protein further comprise a step of assigning a course of treatment or administering a treatment to the subject. Generally, this course of treatment will be assigned when the level of anti-amyloid antibodies in the biological sample is above a control threshold (*e.g*., a threshold indicating a high likelihood or progression of the disease), or more closely resembles a positive control (*e.g*., a control level from a group or individual having experienced progression of the disease) than to a negative control (*e.g*., a control level from a group or individual not having experienced progression of the disease). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g*., Memantine), and a psychiatric drugs (*e.g*., antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In a related embodiment, the present invention provides a method for providing a prognosis for treatment of a disease associated with an amyloid protein, for example a known therapy or preventative strategy for AD. In some embodiments, the treatment can comprise administration of an immunoglobulin preparation such as IVIG in a subject. In some embodiments, the method comprises the steps of (a) contacting the biological sample with a thiophilic resin to bind the anti-amyloid antibody; (b) eluting the antibody from the thiophilic resin at a non-denaturing pH to form an eluate; (c) contacting the eluate with an amyloid antigen to form a complex comprising the amyloid antigen and the anti-amyloid antibody; (d) detecting the presence or level of the complex; and (e) providing a prognosis for treatment of the disease by comparing the level of the anti-amyloid antibody to a control level.

In some embodiments of the methods for providing a prognosis for treatment of a disease associated with an amyloid protein, the control is from an individual or group of individuals that responded favorably to a particular treatment, such that a similar level of the remaining complex relative to control is indicative of a good prognosis for the treatment of the disease. In some embodiments, the control is from an individual or group of individuals that did not responded favorably to a particular treatment, such that a similar level of the remaining complex relative to control is indicative of a poor prognosis for the treatment of the disease. One of skill will understand how to select at least one appropriate control and interpret the results accordingly.

In certain embodiments, the methods for providing a prognosis for treatment of a disease associated with an amyloid protein further comprise a step of assigning a course of treatment or administering a treatment to the subject. Generally, this course of treatment will be assigned when the level of anti-amyloid antibodies in the biological sample more closely resembles a positive control (*e.g*., a control level from a group or individual that responded favorably to a particular treatment) than to a negative control (*e.g*., a control level from a group or individual that did not responded favorably to a particular treatment). In a preferred embodiment, the treatment comprises administration of an immunoglobulin preparation.

In certain embodiments, the treatment is selected from a cognitive treatment, such as physical and/or social activity, memory games, and puzzle and/or problem solving; a pharmaceutical therapy, such as a cholinesterase inhibitor (to address reduced acetylcholine), a partial glutamate antagonists (*e.g*., Memantine), and a psychiatric drugs (*e.g*., antipsychotics, sleep aides, anxiolytics, and beta-blockers). Cholinesterase inhibitors include, without limitation, Aricept® (donepezil hydrochloride), Exelon® (rivastigmine), Razadyne® (galantamine), and Cognex® (tacrine), and combination thereof. In a preferred embodiment, the treatment comprises the administration of an immunoglobulin preparation. In a specific embodiment, the method comprises diagnosing Alzheimer's disease.

In one embodiment of the methods for providing a prognosis for the progression of a disease associated with an amyloid protein or for providing a prognosis for treatment of a disease associated with an amyloid protein, the biological sample is a biological fluid, for example, blood, plasma, urine, lymph, synovial fluid, *etc.* In other embodiments, the biological sample may be a tissue sample or biopsy. In a preferred embodiment, the biological sample is a blood sample or fraction thereof (*e.g*., plasma or plasma fraction). In another preferred embodiment, the biological sample is an immunoglobulin preparation or enrichment. In a specific embodiment, the immunoglobulin enrichment is performed by thiophilic chromatography. In another specific embodiment, the immunoglobulin enrichment is performed by mixed mode ligand chromatography.

In one embodiment, the anti-amyloid antibody being detected is an antibody specific for an amyloid protein selected from Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat. In one embodiment, the anti-amyloid antibody is specific for amyloid protein monomers. In another embodiment, the anti-amyloid antibody is specific for amyloid protein oligomers (*i.e.,* dimers, trimers, *etc*)*.* In yet another embodiment, the anti-amyloid antibody is specific for amyloid protein fibrils.

In one embodiment, the disease or condition associated with an amyloid protein is selected from the group consisting of Alzheimer's disease, Type 2 diabetes mellitus, Parkinson's disease, Transmissible spongiform encephalopathy, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), and systemic AL amyloidosis. In a preferred embodiment, the disease or condition is Alzheimer's disease. In a particularly preferred embodiment, the disease or condition is Alzheimer's disease and the anti-amyloid antibodies being detected are anti-Beta amyloid (Aβ; Abeta) antibodies.

In certain embodiments of the methods provided herein, detection of the presence or level of a particular amyloid protein is useful to provide a prognosis for the progression of a disease or provide a prognosis for the treatment of a disease associated with an amyloid protein. Non-limiting examples of amyloid-amyloid disease combinations that are well suited for use in the methods provided herein can be found in Table 1. In certain embodiments, the detection of the presence or level of a high-avidity antibody specific for the amyloid protein listed in Table 1 will be diagnostic of the corresponding disease listed.

In a preferred embodiment, the anti-amyloid antibody being detected is an antibody specific for Beta amyloid (Aβ; Abeta). In one embodiment, the anti-Aβ antibody is specific for Aβ monomers. In another embodiment, the anti-Aβ antibody is specific for Aβ oligomers (*i.e.,* dimers, trimers, *etc*). In yet another embodiment, the anti-Aβ antibody is specific for Aβ fibrils.

In one embodiment of the methods provided herein, the low-affinity or non-specific antibody is a low avidity anti-amyloid antibody. In a specific embodiment, the low avidity anti-amyloid antibody is a low avidity anti-Aβ antibody.

In certain embodiments of the methods provided herein, the complex comprising the amyloid antigen and the anti-amyloid antibody is washed with a solution containing a chaotropic salt prior to detecting the presence or level of said complex in order to dissociate interactions between non-specific antibodies (*i.e*., low avidity antibodies) and the amyloid antigen. The chaotropic wash step employed in the methods provided herein may be performed with any known chaotropic agent. When a chaotropic salt is employed, the salt will generally comprise either an anion having a chaotropic effect equal or greater than a chlorate ion (ClO₃⁻) or a cation having a chaotropic effect greater than a calcium ion. In one embodiment, the chaotropic agent is selected from urea, thiourea, a guanidinium salt, a thiocyanate salt, a perchlorate salt, an iodide salt, and a chlorate salt. In a specific embodiment, the chaotropic salt is ammonium thiocyanate. In one embodiment, ammonium thiocyanate is used at a wash concentration between about 0.5 M and about 4.0 M. In another embodiment, ammonium thiocyanate is used at a wash concentration between about 1.0 M and about 3.0 M. In yet another embodiment, ammonium thiocyanate is used at a wash concentration between about 1.5 M and about 2.5 M.

The concentration of the chaotropic agent used in the wash step of methods provided herein should be selected such that it is sufficient to disrupt interactions between non-specific (*i.e*., low avidity) anti-amyloid antibodies and amyloid antigens, yet does not disrupt interactions between specific (*i.e*. high avidity) anti-amyloid antibodies and amyloid antigens. The absolute concentration of chaotropic agent employed will depend upon a number of factors, including, the strength of the particular chaotropic agent and the strength of the antibody-amyloid antigen interactions formed in the particular system being employed (*e.g*., anti-Aβ antibody ELISA assay). The skilled artisan will know how to readily determine the optimal concentration of chaotropic agent to use for their particular system. In one embodiment, the chaotropic agent is used at a wash concentration between about 0.5 M and about 4.0 M. In another embodiment, chaotropic agent is used at a wash concentration between about 1.0 M and about 3.0 M. In yet another embodiment, chaotropic agent is used at a wash concentration between about 1.5 M and about 2.5 M.

### Pharmaceutical Compositions and Dosages

A pharmaceutical composition comprising immunoglobulin, *e.g*. an enriched immunoglobulin preparation comprising heterogenous human antibodies can be administered by a variety of methods known in the art. The route and/or mode of administration vary depending upon the desired results, but will typically be intravenous, intramuscular, intraperitoneal, or subcutaneous. The pharmaceutical composition can include an acceptable carrier suitable for intravenous, intramuscular, subcutaneous, parenteral, spinal or epidermal administration (*e.g*., by injection or infusion).

Proper fluidity of the composition can be maintained, for example, by use of coating such as lecithin, by maintenance of required particle size in the case of dispersion and by use of surfactants. In some cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

Pharmaceutical compositions of the invention can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J.R. Robinson, ed., Marcel Dekker, Inc., New York, 1978. Pharmaceutical compositions are preferably manufactured under GMP conditions. Typically, a therapeutically effective dose or efficacious dose of the immunoglobulin preparation is employed in the pharmaceutical compositions of the invention. The pharmaceutical composition can be formulated into dosage forms by conventional methods known to those of skill in the art. Dosage regimens are adjusted to provide the optimum desired response (*e.g*., a therapeutic response). For example, a single bolus may be administered, several divided doses may be administered over time or the dose may be proportionally reduced or increased as indicated by the exigencies of the therapeutic situation. It can be advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels can be varied so as to obtain an amount of the active ingredient which is effective to achieve the desired therapeutic response for a particular patient without being toxic to the patient. A physician can start doses of the pharmaceutical composition at levels lower than that required to achieve the desired therapeutic effect and gradually increase the dosage until the desired effect is achieved. In general, effective doses vary depending upon many different factors, including the specific disease or condition to be treated, its severity, physiological state of the patient, other medications administered, and whether treatment is prophylactic or therapeutic. An exemplary treatment regime entails administration once per every two weeks or once a month or once every 3 to 6 months.

The composition can be administered on multiple occasions. Intervals between single dosages can be weekly, monthly or yearly. Intervals can also be irregular as indicated by measuring therapeutic progress in the patient. Dosage and frequency can vary depending on the half-life of the antibodies in the patient.

In the case of an immunoglobulin preparation, intravenous immunoglobulin (IVIG) is commonly used. The IVIG formulation is designed for administration by injection. Because the IgG preparation of this invention has achieved an exceptionally high immunoglobulin concentration (10% w/v or higher), which significantly reduces the volume for a therapeutically effective dose, the composition of the present invention are particularly advantageous for subcutaneous and/or intramuscular administration to a patient, as well as more commonly used intravenous administration.

The term "effective amount" refers to an amount of an immunoglobulin, particularly IgG, preparation that results in an improvement or remediation of a medical condition being treated in the subject (*e.g*., Alzheimer's disease, Parkinson's disease, *etc.*)*.* An effective amount to be administered to the subject can be determined by a physician with consideration of individual differences in age, weight, disease severity, route of administration (*e.g*., intravenous v. subcutaneous) and response to the therapy. In certain embodiments, an immunoglobulin preparation of this invention can be administered to a subject at about 5 mg/kilogram to about 2000 mg/kilogram each day. In additional embodiments, the immunoglobulin preparation can be administered in amounts of at least about 10 mg/kilogram, at last 15 mg/kilogram, at least 20 mg/kilogram, at least 25 mg/kilogram, at least 30 mg/kilogram, or at least 50 mg/kilogram. In additional embodiments, the immunoglobulin preparation can be administered to a subject at doses up to about 100 mg/kilogram, to about 150 mg/kilogram, to about 200 mg/kilogram, to about 250 mg/kilogram, to about 300 mg/kilogram, to about 400 mg/kilogram each day. In other embodiments, the doses of the immunoglobulin preparation can be greater or less. Further, the immunoglobulin preparations can be administered in one or more doses per day. Clinicians familiar with the diseases treated by IgG preparations can determine the appropriate dose for a patient according to criteria known in the art.

In certain embodiments, a concentrated IgG preparation can be administered to a subject at dose of about 5 mg/kilogram to about 2000 mg/kilogram per administration. In certain embodiments, the dose may be at least about 5 mg/kg, or at least about 10 mg/kg, or at least about 20 mg/kg, 30 mg/kg, 40 mg/kg, 50 mg/kg, 60 mg/kg, 70 mg/kg, 80 mg/kg, 90 mg/kg, 100 mg/kg, 125 mg/kg, 150 mg/kg, 175 mg/kg, 200 mg/kg, 250 mg/kg, 300 mg/kg, 350 mg/kg, 400 mg/kg, 450 mg/kg, 500 mg/kg, 550 mg/kg, 600 mg/kg, 650 mg/kg, 700 mg/kg, 750 mg/kg, 800 mg/kg, 850 mg/kg, 900 mg/kg, 950 mg/kg, 1000 mg/kg, 1100 mg/kg, 1200 mg/kg, 1300 mg/kg, 1400 mg/kg, 1500 mg/kg, 1600 mg/kg, 1700 mg/kg, 1800 mg/kg, 1900 mg/kg, or at least about 2000 mg/kg.

In accordance with the present invention, the time needed to complete a course of the treatment can be determined by a physician and may range from as short as one day to more than a month. In certain embodiments, a course of treatment can be from 1 to 6 months.

### Kits

The invention further provides kits for the detection and/or isolation of high affinity anti-amyloid antibodies. The kits can be used for diagnosis of amyloid-related disorders, and selection of an individual for appropriate therapy, such as with IVIG.

Kits will typically include instructions for use in written or electronic format, and standard reagents, solutions and buffers for the desired assay. The kit can optionally include standard controls or consumable labware, such as ELISA plates, chromatography tools, containers, and reaction vessels. The kit can also include devices for collection of a biological sample, *e.g*., syringes and blood fractionation devices.

The kits of the invention can include materials for the detection of endogenous, high-avidity antibodies that are specific for amyloid. The kit can include thiophilic chromatography reagents and appropriate wash and elution buffers to separate IgG from other proteins in the sample.

The kit can also include materials to separate high avidity amyloid-specific antibodies from low avidity, or non-specific antibodies. Such materials can include a solid support conjugated to the desired form of amyloid, *e.g*., monomeric, oligomeric (globular), or fibrillar amyloid (*e.g*., Aβ). The solid support can be a bead, a chromatography stationary phase (*e.g*., agarose, silica, etc.), an ELISA plate, etc. The materials can also include at least one chaotropic wash buffer, to separate and remove low affinity antibodies from the amyloid-conjugated support. The kit can advantageously include a known high avidity (positive) and low avidity (negative) controls, for comparison to the test sample.

The kits of the invention can further comprise reagents for detection of high avidity anti-amyloid antibodies, as described herein.

In one embodiment, the present invention provides a method for preparing a biological sample for detecting a high avidity anti-amyloid antibody, the method comprising the steps of: (a) contacting a biological sample from the subject with a plurality of amyloid antigens under suitable conditions to form: (i) a complex comprising an amyloid antigen and a low-affinity or non-specific antibody; and (ii) a complex comprising an amyloid antigen and a high avidity anti-amyloid antibody; and (b) washing the amyloid antigen complexes formed in step (a) with a solution containing a chaotropic agent to dissociate at least one complex comprising an amyloid µantigen and a low-affinity or non-specific antibody. In a specific embodiment, the biological sample is an immunoglobulin composition prepared by thiophilic chromatography. In another specific embodiment, the non-specific antibody is a low avidity anti-amyloid antibody.

In one embodiment, the present invention provides a method for preparing a biological sample for detecting a high avidity anti-amyloid antibody, the method comprising the steps of: (a) contacting the biological sample with a thiophilic resin to bind the anti-amyloid antibody; (b) eluting the antibody from the thiophilic resin at a non-denaturing pH to form an eluate; and (c) contacting the eluate with an amyloid antigen to form a complex comprising the amyloid antigen and the anti-amyloid antibody. In a specific embodiment, the method further comprises a step of washing the complex formed in step (c) with a solution containing a chaotropic agent.

In one embodiment, the present invention provides a kit for detecting a high avidity anti-amyloid antibody present in a biological sample, which comprises at least one selected from the group consisting of a chaotropic agent and a thiophilic chromatographic adsorbant. In one embodiment, the kit comprises both a chaotropic agent and a thiophilic chromatographic adsorbant.

In another embodiment, the present invention provides the use of a chaotropic agent and/or a thiophilic chromatographic adsorbant for the manufacture of a kit for detecting a high avidity anti-amyloid antibody present in a biological sample.

In another embodiment, the present invention provides the use of a chaotropic agent and a thiophilic chromatographic adsorbant in combination for the manufacture of a kit for detecting a high avidity anti-amyloid antibody present in a biological sample.

### Examples

### Example 1

Collection of human plasma and IgG preparation. Blood samples from healthy adults and patients with AD were obtained under protocols approved by the Weill Medical College Institutional Review Board and collected in EDTA tubes for the production of plasma by standard methods. Experiments using whole plasma were carried out on specimens stored at -80°C and thawed once by exposure to room temperature for 30 minutes. IgG was purified from plasma by two methods: (1) standard chromatography on Protein G Sepharose (Akerström et al., J Biol Chem., 261:10240-10247 (1986) and (2) preparative chromatography on Thiophilic Absorbant, as specified by the manufacturer (Pierce, Rockford, IL). For Protein G chromatography, IgG was eluted with 0.1M glycine-HCl, pH 2.8, and collected in tubes containing 2M Tris base to reduce time of exposure to low pH. Eluted IgG from either column was dialyzed against PBS prior to use.

### Example 2

Preparation of various oligomeric species of Aβ. Aβ monomer (Biosource International, Camarillo, CA) was dissolved in 50% acetonitrile, incubated for 30 minutes at 37°C and lyophilized. The resulting pellet was dissolved in 1,1,1,3,3,3-hexafluoro-2-propanol, HFIP, incubated at 37°C for 20-30 minutes and then dried under argon.

Aβ oligomers were prepared as previously described (Barghorn et al., J Neurochem., 95:834-847 (2005)). The dried Aβ peptide film was then dissolved at 5 mM in dry DMSO and sonicated in a bath sonicator for 30 minutes to ensure complete disruption of Aβ structures. The Aβ was adjusted to 400 µM with PBS, 1/10^{th} volume of 2% SDS was added and the mixture was incubated at 37°C for 6 hours. The mixture was then diluted with 3 volumes of water and incubated for 18 hours at 4°C. The resultant globular oligomers were characterized by SDS:PAGE and SEC and stored at 4°C for up to four weeks.

Aβ fibrils were prepared by a modification of the method of O'Nuallain (O`Nuallain et al., J Immunol., 176:7071-7078 (2006)), HFIP-treated Aβ monomer was dissolved in freshly made 2 mM NaOH. After gentle agitation, the solution was centrifuged at 10,000 x g for 60 min to remove large clumps or fibrils. The supernatant was then adjusted to 1 x PBS containing 0.05% sodium azide and incubated with agitation at 37°C for 10 to 14 days. Fibril structure was confirmed by transmission electron microscopy of negatively stained grids.

### Example 3

ELISA of human anti-Aβ antibodies. Maxisorp ELISA plate (Nunc, Roskilde, Denmark) were coated with 0.1 ml of 0.1M, pH 9.6, NaHCO₃ buffer containing 0.1 µg total Aβ, either monomer (1 mg/ml in formic acid) or premade Aβ globulomer for 1 hour at 37°C. Aβ fibril plates were made as previously described (O'Nuallain et al., J Immunol., 176:7071-7078 (2006)). The plates were used immediately or stored at 4°C. After removing Aβ coating buffer, the plates were washed 3 times with 0.2 ml of PBS (137 mM NaCl, 2.7 mM KCl, 10 mM Na₂HPO₄, pH 7.4) containing 0.05% Tween 20 (PBST). After the last wash, the plates were blocked with 0.2 ml/well of 1% BSA in PBST for 1 hour at 37°C. The blocking solution was removed and the plates were washed 3 times with PBST as described above.

Each ELISA plate was used to assay 10 plasma samples, diluted 1:10 with PBST, respectively, as well as a negative PBST control and a positive control of IVIG at 10 mg/ml (Gammagard Liquid, Baxter). When complete titers were done, each sample was analyzed as serial three fold dilutions in PBST. The plates were incubated for 1 hr at 37°C, washed 3 times with 0.2 ml PBST, and incubated with 0.1 ml of a 1:10,000 dilution of horseradish peroxidase-conjugated goat, affinity-purified anti-human IgG antibody (Biosource International, Camarillo, CA) in PBST for 30 minutes at 37°C. The plates were washed 4 times with PBST and developed by adding 0.1 ml/well TMB (Invitrogen, Carlsbad, CA) for 15-30 minutes at room temperature. At the first indication that the negative control wells were changing color, 0.1 ml of 1M HCl was added to each well to terminate the reaction. The OD at 405 nm was measured using a Synergy HT ELISA reader (Bio-Tek, Winooski, VT).

The anti-Aβ antibody titers were calculated using the KC4 Signature software to fit a four parameter sigmoid curve to the ODs obtained from the serial dilutions of plasma or IVIG. Prior to curve fitting, the average OD of the secondary antibody-only controls was subtracted from the OD of wells with plasma, or IVIG. If necessary, the dilution of the sample was reduced so that the maximum OD of the least dilute sample was less than the half-max of the IVIG standard. The titer of the anti-Aβ antibody in a sample was taken as the reciprocal of the dilution at which the OD was equal to one-half the maximum OD (usually 2.0-3.0) of the IVIG standard diluted to 10 mg/ml.

### Example 4

SPR measurements. A carboxylated sensor was loaded into a SensiQ SPR apparatus ICX Nomadics, Oklahoma City, OK), rinsed with a 1 minute injection of 0.1M phosphoric acid to remove surface contaminants and activated by injection of 2 mM EDC, 0.5 mM NHS into both control and experimental channels. Neutravidin (25 µg/ml) in 10 mM acetate buffer pH 4.7 was injected into the experimental channel followed by a 50 µg/mL solution of biotin-tagged Aβ monomer, oligomer or fibril in 10 mM HEPES buffer (pH 7.4), 150 mM NaCl, 1.34 mM EDTA, and 1% Tween 20. Coupling was terminated by the injection of 1M ethanolamine (pH 8) into both channels. SPR antibody binding assays were done using serial two-fold dilutions of plasma or purified IgG preparations. Analysis of binding was done using Qdat software (ICX Nomadics, Oklahoma City, OK).

### Example 5

Selective antigen-antibody dissociation by chaotrophic salt. Conditions for selective dissociation were determined by first measuring the avidity of antibodies binding to blank plates and Aβ-coated plates. We then determined the molarity of the chaotropic salt (ammonium thiocyanate) necessary to provide the optimum dissociation between binding to blank or amyloid plates (Pullen et al., J Immunol Methods., 86:83-87 (1986)). Briefly, an Aβ-coated ELISA plate was incubated with 1:10 dilution of human plasma or murine monoclonal anti-Aβ antibodies (1 ug/ml) diluted in PBST as described above. The wells were emptied and 0.2 ml of 50 mM Na phosphate buffer, pH 6.0, or this buffer containing increasing concentrations of ammonium thiocyanate was added in quadruplicate to each set of wells. The plates were incubated with for 30 minutes at 25°C, washed three times with PBST and processed as described above for standard ELISA.

### Example 6

Isolation of Anti-amyloid antibodies by Affinity Purification. Resins for affinity purification of anti-Aβ monomer antibodies that couple Aβ peptide to the resin at the amino or carboxyl terminus were obtained commercially (Alpha Diagnostic Intl., San Antonio, TX). Resins for anti-Aβ oligomer and anti-Aβ fibril antibodies were prepared by coupling Aβ oligomers and fibrils, made as described above, to N-hydroxysuccinimide-activated Sepharose 4 Fast Flow as recommended by the manufacturer (GE Healthcare, Uppsala, Sweden). The columns were equilibrated in PBS and 5 g/ml IVIG diluted to 10 mg/ml in PBS was passed over the columns at 0.5 ml/min for a total of at least 5 complete passes of 500 ml before washing with PBS and standard elution with 0.1 M glycine, pH 2.5. The eluted IgG was immediately neutralized, concentrated by centrifugation on ultrafiltration spin columns and dialyzed against PBS.

### Example 7

Blank plate binding during anti-Aβ antibody measurements. Quantification of anti-Aβ antibodies in human plasma or serum has been carried out in most instances on ELISA plates bearing either monomeric or aggregated (oligomeric or fibrillar) Aβ peptide. Plasma samples from normal human donors assayed by this method bind significantly to blank ELISA wells (wells not bearing any added antigen). Extensive, non-specific blank plate binding occurs despite the use of traditional blocking agents such as skim milk, fetal calf serum, albumin and commercial blocking preparations (Klaver et al., J. Neurosci. Meth. 187, 263-269). The extent of binding to blank ELISA plates is variable across plasma donors (Figure 1).

Binding to blank ELISA plates also occurs with purified IgG isolated from plasma samples and pooled immunoglobulin preparations such as IVIG. IVIG binds in a saturable, concentration-dependant fashion to blank, Aβ monomer, Aβ oligomer and Aβ fibril plates. The maximum binding values in A_{450 nm} units was 4.54 (blank), 2.7 (monomer), 2.88 (oligomer), and 2.82 (fibril), respectively. Based on the best fit of the ELISA curves, the maximum binding for IVIG in absorbance units is approximately 1.6-fold greater on blank plates than on any of the amyloid bearing plates. Similar results were obtained for IgG isolated from plasma of normal individuals or AD patients. The absolute values of the maximum binding differed depending on the individual. The substantial binding of IVIG to the blank plate indicates a greater number of potential antibody binding sites on the blank plates relative to the amyloid-bearing plates.

Despite the difference in maximum binding amplitude, the apparent titers of antibodies binding to blank ELISA wells were generally lower than those binding to wells containing Aβ peptides or its assemblies. Blank plate half maximum binding titers for IVIG were 17.6-, 3.9- and 6.5-fold lower than that for the anti-Aβ monomer, Aβ oligomer and Aβ fibril antibodies, respectively. Typical half maximum titer values were 2.44 (blank plate), 42.84 (monomer), 9.42 (oligomer), and 15.85 (fibril). Similar results were obtained with IgG isolated from the plasma of most individual donors. The extent of blank plate binding varied across individuals but the maximal binding values derived from fitting the ELISA curves were always higher than for blank plates than the anti-amyloid antibodies. This suggests that measurements of anti-amyloid antibody levels based on a single dilution rather than a titer curve are likely to be over-estimations because of the added effects of blank plate binding.

The presence of antibodies that have the capacity to bind to blank ELISA wells complicates measurement of anti-Aβ oligomer antibodies in individuals since the observed absorbance values obtained for empty wells can be comparable or even greater than those obtained for amyloid-bearing plates (Figure 1). This does not mean that the plate-binding antibodies are identical. In fact, the absorbance obtained with empty wells at the concentrations used were greater than that for wells containing amyloid in six of the twenty-two individuals shown in Figure 1 suggesting that the antibodies do not all have the same specificities. Blank plate binding may thus reflect the presence of polyvalent antibodies that in some cases are capable of binding to both blank plates and amyloid-bearing plates.

### Example 8

Blank plate binding is Fab mediated. To determine the nature of the plate binding interaction, we examined binding of a commercially available pool of IgG's from 5 individuals, as well as F_{ab}, F_{ab}2', and F_{c} fragments isolated from this same pool. We found that binding of antibodies to blank ELISA wells was the result of binding to the antigen-binding region (F_{ab}) of the IgG molecule, as opposed to non-specific interactions involving other domains. Figure 2 shows the antigen-binding region (F_{ab} and F_{ab}2' fragments) of IgG is responsible for the binding to the empty wells rather than a non-specific interaction through other domains of the immunoglobulin molecule.

### Example 9

Depletion of blank plate binding antibodies. Isolation of IgG from plasma samples does not eliminate the binding of antibodies to blank ELISA plates. It was tested whether passage of IVIG over columns of polystyrene and/or agarose would deplete IgG molecules that readily bound to these substrates and possibly reduce the binding of IVIG to blank ELISA wells. The depletion was not specific for blank plate binding antibodies. The absolute ratio of the absorbance obtained with blank plates and Aβ-bearing plates remained relatively constant regardless of column chromatography. A further drawback to this approach was that the total amount of anti-Aβ-binding antibody was substantially reduced by all of the depletion procedures, suggesting that some of the antibodies that bind to Aβ can also adhere to agarose or polystyrene. Such binding is most likely to be attributable to low affinity, polyvalent antibodies that bind to multiple, unrelated epitopes, including Aβ and polystyrene.

More direct evidence for polyvalency of certain anti-Aβ antibodies comes from a competition study using the plasma protein, thyroglobulin (Figure 3). The binding of IVIG to Aβ peptide-bearing ELISA wells was reduced when thyroglobulin was used as the competitor, confirming that a large pool of anti-Aβ antibodies in IVIG are polyvalent and can bind to both of these proteins. As seen in Figure 3, a concentration of thyroglobulin which completely eliminated binding of IVIG to thyroglobulin-bearing ELISA plates reduces the binding of IVIG to Aβ plates to approximately 50% of control values.

### Example 10

Reduction of blank plate binding by chaotropic salt. The potential to separate antibodies that bound to blank wells from those bound to Aβ based on differences in avidity was examined. Avidity measurements were performed using increasing concentrations of the chaotropic salt ammonium thiocyanate to dissociate antibody/antigen complexes formed on ELISA plates. The binding of IVIG to ELISA plates whose wells were either blank or were coated with globular Aβ oligomer were compared. Figure 4 shows a typical experiment indicating that the avidity of anti-globular Aβ oligomer antibodies in IVIG is clearly greater than that seen for the binding of IVIG to blank plates. On the blank plates, 50% of the binding seen for phosphate buffer control conditions occurs when plates are incubated in approximately 2.5M ammonium thiocyanate. For the Aβ oligomer-bearing plates, reduction to 50% binding is not achieved even at 4M ammonium thiocyanate, the highest concentration used in this study. Aβ oligomers prepared by several independent methods, including cross-linked and non cross-linked methods (Barghom et al., J Neurochem., 95:834-847 (2005); Kayed et al., Molecular Neurodegeneration, 2:18-29 (2007)), gave essentially identical results.

These results suggest that at least some anti-Aβ oligomer antibodies can be differentiated from antibodies that bind to blank plates on the basis of binding avidity. To further test this hypothesis, we depleted IVIG of high affinity anti-Aβ oligomer antibodies by passage over an affinity column bearing Aβ globulomers. For the anti-Aβ oligomer-depleted IVIG, the concentration of chaotropic salt required to achieve 50% maximal binding of globular Aβ oligomer was reduced from >4M to 1.3M ammonium thiocyanate. Similarly, there was a reduction from 2.5M to 0.9M ammonium thiocyanate for empty wells. These results suggest that some antibodies in the human repertoire interact with both Aβ oligomers and empty wells. However there are also antibodies that have a high avidity for Aβ oligomers specifically.

### Example 11

To confirm that incubation in ammonium thiocyanate following antibody binding would allow discrimination between high affinity anti-Aβ oligomer antibodies and those that bind more promiscuously to empty ELISA wells, the avidity of IgG in plasma and purified IgG from four individuals, two young and two elderly controls was determined (Figure 5). Purified IgG, at 1 mg/ml, from these individuals exhibited consistent binding to empty wells. The observed absorbance for blank plate binding is reduced by ∼80% following 4M thiocyanate treatment; similar to the result obtained with IVIG in Example 10. In contrast, the relative binding to wells bearing Aβ42 oligomers was reduced by only ∼20%. As with Aβ oligomer, both anti-Aβ monomer and anti-Aβ fibril antibodies are more resistant to ammonium thiocyanate dissociation than antibodies binding to blank plates. Thus, it appears possible to define a set of conditions that permits the measurement of anti-Aβ antibodies in mixtures of purified human antibodies with minimal interference from antibodies that have the capacity to bind to empty ELISA wells.

### Example 12

Generation of polyvalent antibodies by acids and destabilizing agents. Exposure of serum to even mild acidic conditions has been shown to increase the titers of polyvalent antibodies, many of which target auto-antigens (Bouvet et al., J Autoimmun., 16:163-172 (2001); Djoumerska et al., Scand J of Immunol., 61: 357-363 (2005)). The generation of polyvalency by mild acidification has also been reported for a specific monoclonal antibody in which the pH change resulted in increased polyvalent binding through a partial denaturation of antigen binding site (Dimitrov et al., Molec Immunol, 44:1854-1863 (2007)). We tested several lots of IVIG (Gammaguard, Baxter) and found that acidification at pH 3.0 led to an increase in Aβ monomer titers, ranging from 3.7- to 9.8-fold. This increase in the titer was reversed by a 24 hour incubation period at neutral pH and room temperature. Generation of polyvalency by incubation at pH 2.0 was not fully reversible. This suggests that incubation at very low pH irreversibly alters the structural integrity of antibodies, which nevertheless retain the capacity to bind non-specifically to a variety of antigens.

Exposure to 6 M urea, a more stringent denaturation treatment, also increases the apparent titer of a number of auto-antibodies present in IVIG (Bouvet et al., J Autoimmun., 16:163-172 (2001)). IVIG at 10 mg/ml in PBS was dialyzed against PBS alone or with 6M urea. The increase in antibody titer was calculated by dividing the antibody titer after urea treatment by the antibody titer of the PBS control (Wardemann et al., Science, 301:1374-1377 (2003). The results shown in Table 1 are an average of a minimum of two experiments for each autoantigen. Table 1 shows that 6M urea treatment increased binding of antibodies in IVIG to a set of auto-antigens including Aβ. The increases ranged from 4- to 23-fold after urea treatment compared to control IVIG preparations, with the greatest effect seen for Aβ peptide. Therefore, partial denaturation of IgG particularly increases the titer of serum anti-Aβ antibodies, presumably by altering the antigen binding site to allow more promiscuous binding.

**Table 1. Increase in Autoantibody Titer in IVIG Following Dialysis against 6M Urea***

| **Auto-antigen** | **Fold Increase in Titer** |
|---|---|
| Aβ42 Peptide Monomer | 23 |
| Thyroglobulin | 9 |
| LPS+ | 4 |
| ssDNA+ | 6 |
| dsDNA+ | 5 |
| Insulin+ | 5 |

The artifactual generation of Aβ binding activity by exposure to acids or other denaturing conditions has important implications for the measurement of anti-Aβ antibody titers in purified human IgG preparations. For example, in normal controls the half-max titer for blank plate binding by IgG purified by the Protein G method, averaged > than 100-fold higher than that for the original plasma. In these same individuals, a 50-fold increase was seen after acidification for binding to oligomer plates. Purification of human IgG by methods that can at least partially denature IgG, *e.g*., acid elution from Protein G resins, results in artificially high titers due to acid-induced polyvalency.

### Example 13

Effects of non-denaturing IgG purification. We then investigated whether isolation of IgG by thiophilic absorbant chromatography would avoid the creation of polyvalency associated with acid elution. Thiophilic absorbent chromatographic exploits the binding of IgG to a moiety in which a sulphone group is in close proximity to a thioether group. As shown Figure 6, IgG prepared by Thiophilic chromatography removes a majority of non-immunoglobulin proteins present in plasma. While Thiophilic Absorbant is somewhat less effective in removing other plasma constituents than Protein G chromatography, it does not subject the immunoglobulin to denaturing conditions that can foster generation of polyvalency. Consistent with this assertion, repurification of IgG from IVIG using Thiophilic Absorbant does not alter the titer of antibodies binding to either Aβ or blank plates.

To compare the binding of anti-Aβ antibodies in whole plasma with those purified by various methods, surface plasmon resonance (SPR) was employed as described above. We compared the binding of whole plasma from a normal control to IgG purified from the same plasma by either Thiophilic Absorbant or Protein G chromatography. Measurements were carried out on a Aβ oligomer sensor. The Aβ oligomer sensor comprised a biotinylated Aβ oligomer bound to Neutravidin, which was coupled covalently to PEG chains on the sensor.

SPR response curves for comparable concentrations of the three preparations are shown in Figure 7. IgG purified by thiophilic reagent yields a more positive deflection than whole plasma. This difference likely reflects decreased interference by plasma proteins with Aβ binding. When IgG was isolated by Thiophilic chromatography, the increase in the half-max titer for blank plate binding was approximately 3-fold greater than plasma and that for the binding to oligomer plates almost 5-fold greater. Thus, the specific binding to Aβ oligomer plates is improved with the Thiophilic isolation method, most likely reflecting the elimination of interfering plasma proteins.

The IgG purified by Protein G chromatography showed a strikingly different, negative response that indicates greater binding to the control sensor than the antigen-bearing plate. The control sensor has a coating of PEG with alanine at the terminus, and the negative response after Protein G chromatography most likely results from the creation of new polyvalent specificities in the IgG after acid exposure, at least some of which recognize the PEG coating on the control sensor.

### Example 14

Measurement of plasma anti-amyloid activity. To determine whether anti-amyloid activity measured in human plasma was exclusively the result of natural or induced polyvalency, affinity chromatography was used to separate human anti-amyloid antibodies of various specificities from IVIG, including anti-Aβ monomer, Aβ oligomer and Aβ fibril antibodies. In the case of anti-Aβ monomer antibodies, two types of antibodies were isolated. The first set of antibodies bound to Aβ monomer coupled to the affinity resin at the amino terminus (anti-AβN) and the second bound to Aβ monomer coupled at the carboxyl terminus (anti-AβC).

We determined the dissociation constants for the two types of anti-Aβ monomer and the anti-oligomer antibodies by SPR analysis. Table 2 shows the K_{d} measurements for the anti-amyloid antibodies we isolated, and for 6E10 a monoclonal anti-Aβ antibody, which binds to an Aβ epitope located near the amino terminus. The K_{d} values indicate that the affinity purified antibodies have different specificities, as the binding affinities were higher to sensors containing the same form of amyloid as was used for the affinity purification. There may, however, be some overlapping specificity. All affinity purified antibody preparations showed binding to wells of blank ELISA plates. The percent of the OD seen on the various amyloid plates that was obtained for blank wells binding varied with the different affinity-purified antibody preparations. For instance, in the case of anti-AβN antibody, binding to blank wells was equal to approximately 28% of the binding to monomer wells, 15% of the binding to fibril wells and essentially all of the binding to the oligomer wells.

**Table 2. K_{d}'s of affinity-purified anti-amyloid antibodies from IVIG**

| Sensor | Antibody | K_{d} (nM) |
|---|---|---|
| C-terminal monomer | C-terminal monomer | 650 |
| | N-terminal monomer | 24,000 |
| | Oligomer | 20,000 |
| | 6E10 | 47 |
| N-terminal monomer | C-terminal monomer | 1,900 |
| | N-terminal monomer | 450 |
| | Oligomer | 4,000 |
| | 6E10 | 158 |
| Oligomer | C-terminal monomer | 4,400 |
| | N-terminal monomer | 2,300 |
| | Oligomer | 590 |
| | 6E10 | 69 |

Table 3 summarizes the blank plate binding properties of the affinity purified anti-Aβ antibodies isolated from IVIG. In the case of anti-fibril antibodies, two different preparations were assayed. The first antibody preparation was affinity purified on an Aβ42 fibril column, and includes antibodies to Aβ peptide and structural neo-epitopes associated with fibrillization. The second preparation of anti-Aβ fibril antibody was affinity purified a second time on an IAPP (islet amyloid polypeptide) fibril column, and presumably contains only those antibodies to the neo-epitopes associated with fibrils (O'Nuallain et al., J Immunol., 176:7071-7078 (2006)). The latter fraction showed decreased blank plate binding.

**Table 3. Blank plate binding of affinity purified anti-Aβ antibodies. Ratio of blank plate binding to amyloid plate binding.**

| Affinity-purified antibodies | ELISA Plates | | |
|---|---|---|---|
| | Monomer | Oligomer | Fibril |
| anti-Aβ N monomer | 0.28 | 1.00 | 0.15 |
| anti-Aβ C monomer | 0.56 | 0.36 | 0.49 |
| anti-Aβ oligomer | 0.58 | 0.73 | 0.62 |
| anti-Aβfibril # 1* | 0.72 | 1.00 | 0.62 |
| anti-Aβfibril #2** | 0.27 | 0.63 | 0.13 |

| | | | |
|---|---|---|---|
| * Purified by passage over an fibrillar Aβ42 affinity resin ** Purified over an fibrillar Aβ42 affinity resin followed by repurification on an fibrilar IAPP resin | | | |

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

## Claims

1. A method for detecting a high avidity anti-amyloid antibody present in a biological sample, the method comprising the steps of:
(a) contacting the biological sample with a plurality of amyloid antigens under suitable conditions to form:
(i) a complex comprising an amyloid antigen and a non-specific antibody; and
(ii) a complex comprising an amyloid antigen and a high avidity anti-amyloid antibody;
(b) washing the amyloid antigen complexes formed in step (a) with a solution containing a chaotropic agent to dissociate at least one complex comprising an amyloid antigen and a non-specific antibody; and
(c) detecting the presence or level of the remaining complexes.

2. The method of claim 1, wherein the biological sample is an immunoglobulin composition prepared by thiophilic chromatography.

3. A method for detecting a high avidity anti-amyloid antibody present in a biological sample, the method comprising the steps of:
(a) contacting the biological sample with a thiophilic resin to bind the anti-amyloid antibody;
(b) eluting the antibody from the thiophilic resin at a non-denaturing pH to form an eluate;
(c) contacting the eluate with an amyloid antigen to form a complex comprising the amyloid antigen and the anti-amyloid antibody; and
(d) detecting the presence or level of the complex.

4. The method of claim 3, wherein the method further comprises a step of washing the complex formed in step (c) with a solution containing a chaotropic agent.

5. A method for diagnosing or providing a prognosis for a disease associated with an amyloid protein in a subject, the method comprising the steps of:
(a) contacting a biological sample from the subject with a plurality of amyloid antigens under suitable conditions to form:
(i) a complex comprising an amyloid antigen and a non-specific antibody; and
(ii) a complex comprising an amyloid antigen and a high avidity anti-amyloid antibody;
(b) washing the amyloid antigen complexes formed in step (a) with a solution containing a chaotropic agent to dissociate at least one complex comprising an amyloid antigen and a non-specific antibody;
(c) detecting the level of the remaining complexes; and
(d) diagnosing or providing a prognosis for the subject by comparing the level of the anti-amyloid antibody to a control level.

6. The method of claim 5, wherein the biological sample is an immunoglobulin composition prepared by thiophilic chromatography.

7. A method for diagnosing or providing a prognosis for a disease associated with an amyloid protein in a subject, the method comprising the steps of
(a) contacting the biological sample with a thiophilic resin to bind the anti-amyloid antibody;
(b) eluting the antibody from the thiophilic resin at a non-denaturing pH to form an eluate;
(c) contacting the eluate with an amyloid antigen to form a complex comprising the amyloid antigen and the anti-amyloid antibody;
(d) detecting the presence or level of the complex; and
(e) diagnosing or providing a prognosis for the subject by comparing the level of the anti-amyloid antibody to a control level.

8. The method of claim 7, wherein the method further comprises a step of washing the complex formed in step (c) with a solution containing a chaotropic agent.

9. The method according to any one of claims 5 to 11, wherein the disease is selected from the group consisting of Alzheimer's disease, Type 2 diabetes mellitus, Parkinson's disease, Transmissible spongiform encephalopathy, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), and systemic AL amyloidosis.

10. The method of claim 9, wherein the disease is Alzheimer's disease.

11. The method according to any one of claims 1 to 10, wherein the method further comprises administering a therapeutically effective amount of an immunoglobulin preparation to the subject.

12. The method according to any one of claims 1 to 11, wherein the anti-amyloid antibody is specific for an amyloid protein selected from the group consisting of Beta amyloid (Aβ; Abeta), Islet amyloid polypeptide (IAPP; Amylin), Alpha-synuclein (SNCA), Major Prion Protein (PrP), Huntingtin (HD), Calcitonin (CCP), Atrial natriuretic factor (ANF), Apolipoprotein AI (Apo-A1), Serum amyloid A protein (SAA), Medin amyloid (fragment of Milk fat globule-EGF factor 8 protein; MFG-E8), Prolactin (PRL), Transthyretin (ATTR), Lysozyme C (1,4-beta-N-acetylmuramidase C), Beta 2 microglobulin (β2M), Gelsolin (AGEL), Transforming growth factor-beta-induced protein ig-h3 (Beta ig-h3; Keratoepithelin), Cystatin C (CST3), Immunoglobulin light chain (AL), and an amyloid protein having a polyQ repeat.

13. The method according to any one of claims 1 to 12, wherein the anti-amyloid antibody specifically recognizes amyloid protein monomers.

14. The method according to any one of claims 1 to 12, wherein the anti-amyloid antibody specifically recognizes amyloid protein oligomers.

15. The method according to any one of claims 1 to 12, wherein the anti-amyloid antibody specifically recognizes amyloid protein fibrils.

16. The method according to any one of claims 1 to 15, wherein the anti-amyloid antibody is an anti-beta amyloid antibody.
